# EUROPÄISCHE PATENTSCHRIFT

(11) **EP 0 758 652 B1**
(45) Veröffentlichungstag und Bekanntmachung des Hinweises auf die Patenterteilung: **20.10.2004**
(21) Anmeldenummer: 96112214.0
(22) Anmeldetag: 29.07.1996
(51) Int. Cl.: C07D 487/04, A01N 43/90

(54) **Substituierte Tetrahydro-5-nitro-pyrimidine und deren Verwendung zur Bekämpfung von tierischen Schädlingen**
Substituted tetrahydro-5-nitro-pyrimidines and their use as pesticides
Tétrahydro-5-nitro-pyrimidines et leur application comme pesticides

(30) Priorität: 10.08.1995 DE 19529411
(43) Veröffentlichungstag der Anmeldung: 19.02.1997
(73) Patentinhaber: Bayer CropScience AG, 40789 Monheim (DE)
(72) Erfinder: Gesing, Ernst Rudolf, Dr., 40699 Erkrath (DE); Wolf, Hilmar, Dr., 40764 Langenfeld (DE); Erdelen, Christoph, Dr., 42799 Leichlingen (DE); Wachendorff-Neumann, Ulrike, Dr., 56566 Neuwied (DE); Andersch, Wolfram, Dr., 51469 Bergisch Gladbach (DE); Turberg, Andreas, Dr., 40699 Erkrath (DE); Mencke, Norbert, Dr., 51381 Leverkusen (DE)

(56) Entgegenhaltungen:
- EP-A- 0 247 477
- EP-A- 0 316 843
- EP-A- 0 316 845
- WO-A-94/05670

## Beschreibung

Die vorliegende Erfindung betrifft neue Tetrahydro-5-nitro-pyrimidine, ein Verfahren zu ihrer Herstellung und ihre Verwendung zur Bekämpfung von tierischen Schädlingen, insbesondere von Insekten, Spinnentieren und Nematoden, die in der Landwirtschaft, in den Forsten, im Vorratsund Materialschutz sowie auf dem Hygienesektor vorkommen.

Es ist bereits bekannt, dass bestimmte 1,2,3,6-Tetrahydro-5-nitro-pyrimidin-Derivate insektizide Eigenschaften aufweisen (vgl. DE-OS 36 38 121, EP-A 0 316 843 und EP-A 0 316 845). Die Wirkung dieser Verbindungen ist jedoch, insbesondere bei niedrigen Aufwandmengen und Konzentrationen nicht immer zufriedenstellend.

Es wurden neue substituierte Tetrahydro-5-nitro-pyrimidine der Formel (I) 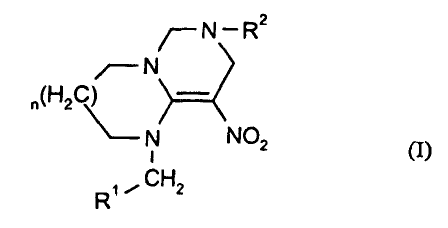 gefunden, in welcher
- n: für 0 oder 1 steht,
- R¹: für gegebenenfalls einfach bis dreifach, gleich oder verschieden substituiertes Pyridyl oder für gegebenenfalls einfach bis zweifach, gleich oder verschieden substituiertes Thiazolyl steht, wobei jeweils als Substituenten in Frage kommen:
Halogen, Cyano, Nitro, C₁-C₄-Alkyl, C₁-C₄-Alkoxy, C₁-C₄-Alkylthio, C₁-C₄-Halogenalkyl, C₁-C₄-Halogenalkoxy und C₁-C₄-Halogenalkylthio,
- R²: für eine der folgenden Gruppierungen steht: 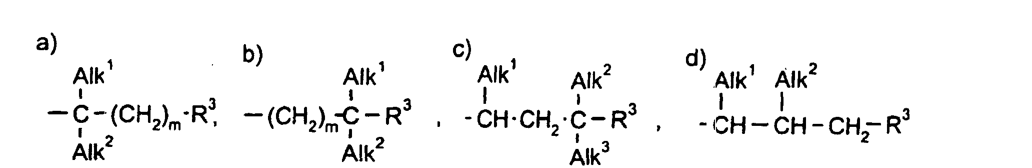 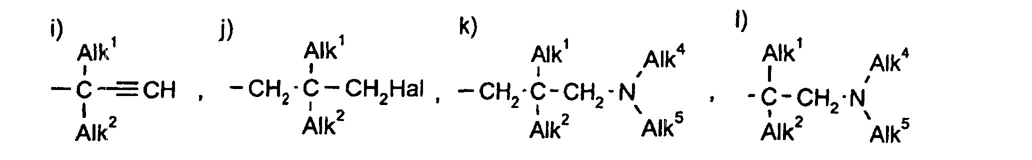 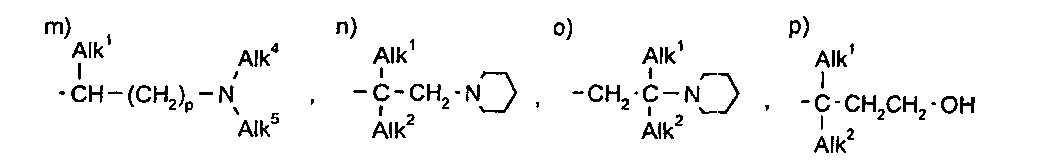 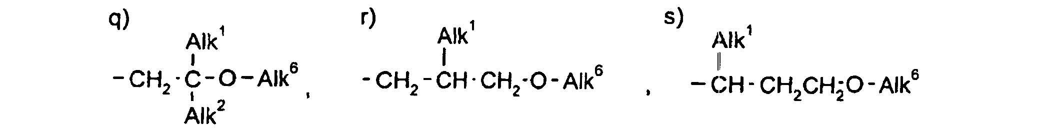 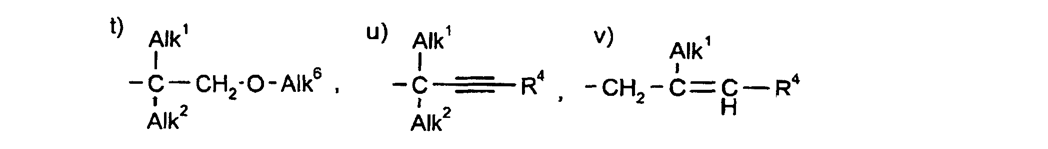 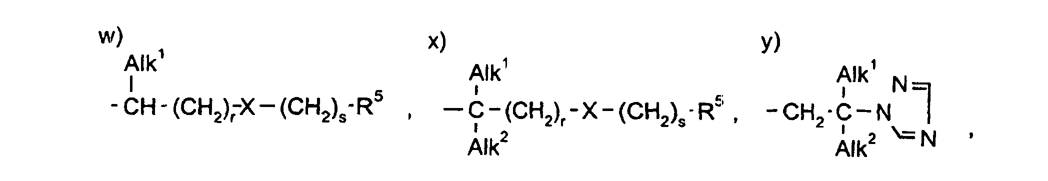
wobei
- Alk¹ bis Alk⁶: unabhängig voneinander für C₁-C₄-Alkyl stehen,
- Hal: für Fluor, Chlor oder Brom steht,
- m: für 1 oder 2 steht,
- p: für 1, 2 oder 3 steht,
- r und s: unabhängig voneinander für 0, 1, 2, 3 oder 4 stehen,
- R³: für gegebenenfalls einfach bis fünffach, gleich oder verschieden substituiertes Phenyl oder für gegebenenfalls einfach bis vierfach, gleich oder verschieden substituiertes Pyridyl steht, wobei jeweils als Substituenten in Frage kommen:
Halogen, Cyano, Nitro, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Amino, C₁-C₄-Alkylamino und Di(C₁-C₄-Alkyl)amino;
sowie für gegebenenfalls einfach bis zweifach, gleich oder verschieden substituiertes N-Morpholino, wobei als Substituenten in Frage kommen:
Halogen, C₁-C₄-Alkyl, C₁-C₄-Alkoxy und C₁-C₄-Halogenalkyl,
- R⁴: für gegebenenfalls einfach bis fünffach, gleich oder verschieden substituiertes Phenyl oder für gegebenenfalls einfach bis dreifach, gleich oder verschieden substituiertes Thienyl steht, wobei jeweils als Substituenten in Frage kommen:
Halogen, Cyano, Nitro, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Amino, C₁-C₄-Alkylamino und Di(C₁-C₄-Alkyl)amino,
- R⁵: für gegebenenfalls einfach bis fünffach, gleich oder verschieden substituiertes Phenyl, gegebenenfalls einfach bis dreifach, gleich oder verschieden substituiertes Naphthyl oder für gegebenenfalls einfach bis vierfach, gleich oder verschieden substituiertes Pyridyl steht, wobei jeweils als Substituenten in Frage kommen:
Halogen, Cyano, Nitro, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Amino, C₁-C₄-Alkylamino und Di(C₁-C₄-Alkyl)amino und
- X: für Sauerstoff, Schwefel oder die Gruppierung NAlk⁷ steht, wobei
Alk⁷ für C₁-C₄-Alkyl steht.

Weiterhin wurde gefunden, dass man die substituierten Tetrahydro-5-nitro-pyrimidine der Formel (I) erhält, wenn man Nitromethylen-Derivate der Formel (II) 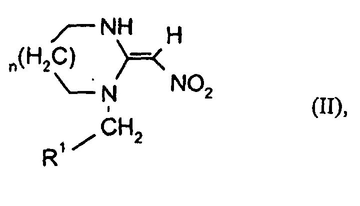 in welcher
- R¹ und n: die oben angegebene Bedeutung haben,
mit Aminen der Formel (III)

H₂N-R² (III),

in welcher
- R²: die oben angegebene Bedeutung hat,
in Gegenwart von mindestens der zweifachen molaren Menge Formaldehyd, gegebenenfalls in Gegenwart eines sauren Katalysators und gegebenenfalls in Gegenwart eines Verdünnungsmittels umsetzt.

Die substituierten Tetrahydro-5-nitro-pyrimidine der Formel (I) besitzen gegebenenfalls asymmetrisch substituierte Kohlenstoffatome und können deshalb in verschiedenen optischen Isomeren anfallen. Die vorliegende Erfindung betrifft sowohl die Isomerengemische als auch die reinen Isomeren.

Schließlich wurde gefunden, dass die neuen substituierten Tetrahydro-5-nitro-pyrimidine der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.

Die erfindungsgemäßen substituierten Tetrahydro-5-nitro-pyrimidine sind durch die Formel (I) allgemein definiert.

Bevorzugte Substituenten bzw. Bereiche der in den oben und nachstehend erwähnten Formeln aufgeführten Reste werden im folgenden erläutert:
- n: steht bevorzugt für 0 oder 1.
- R¹: steht bevorzugt für gegebenenfalls einfach bis zweifach, gleich oder verschieden substituiertes Pyridyl oder für gegebenenfalls einfach substituiertes Thiazolyl, wobei jeweils als Substituenten in Frage kommen:
Fluor, Chlor, Brom, Cyano, Nitro, Methyl, Ethyl, Methoxy, Methylthio, Trifluormethyl, Trifluormethoxy und Trifluormethylthio.
- R²: steht bevorzugt für die Gruppierungen (a) bis (d) und (i) bis (y).
- Alk¹ bis Alk⁶: stehen unabhängig voneinander bevorzugt für Methyl, Ethyl und n- oder i-Propyl.
- Hal: steht bevorzugt für Fluor und Chlor.
- m: steht bevorzugt für 1 oder 2.
- p: steht bevorzugt für 1, 2 oder 3.
- r und s: stehen unabhängig voneinander bevorzugt für 0, 1, 2, 3 oder 4.
- R³: steht bevorzugt für gegebenenfalls einfach bis dreifach, gleich oder verschieden substituiertes Phenyl oder für gegebenenfalls einfach bis zweifach, gleich oder verschieden substituiertes Pyridyl, wobei jeweils als Substituenten in Frage kommen:
Fluor, Chlor, Brom, Cyano, Nitro, Methyl, Ethyl, n- oder i-Propyl, t-Butyl, Trifluormethyl, Methoxy, Ethoxy, Trifluormethoxy, Trifluormethylthio, Dimethylamino, Methylethylamino und Diethylamino;
sowie für gegebenenfalls einfach bis zweifach, gleich oder verschieden substituiertes N-Morpholino, wobei als Substituenten in Frage kommen:
Methyl, Ethyl, Methoxy und Trifluormethyl.
- R⁴: steht bevorzugt für gegebenenfalls einfach bis dreifach, gleich oder verschieden substituiertes Phenyl oder für gegebenenfalls einfach bis zweifach, gleich oder verschieden substituiertes Thienyl, wobei jeweils als Substituenten in Frage kommen:
Fluor, Chlor, Brom, Cyano, Nitro, Methyl, Ethyl, n- oder i-Propyl, t-Butyl, Trifluormethyl, Methoxy, Ethoxy, Trifluormethoxy, Trifluormethylthio, Dimethylamino,
Methylethylamino und Diethylamino.
- R⁵: steht bevorzugt für jeweils gegebenenfalls einfach bis dreifach, gleich oder verschieden substituiertes Phenyl und Naphthyl oder für gegebenenfalls einfach bis zweifach, gleich oder verschieden substituiertes Pyridyl, wobei jeweils als Substituenten in Frage kommen:
Fluor, Chlor, Brom, Cyano, Nitro, Methyl, Ethyl, n- oder i-Propyl, t-Butyl, Trifluormethyl, Methoxy, Ethoxy, Trifluormethoxy, Trifluormethylthio, Dimethylamino, Methylethylamino und Diethylamino.
- X: steht bevorzugt für Sauerstoff, Schwefel, N-Methyl, N-Ethyl, N-n-Propyl und N-i-Propyl.

Die oben aufgeführten allgemeinen oder in Vorzugsbereichen aufgeführten Restedefinitionen bzw. Erläuterungen gelten für die Endprodukte und für die Ausgangs- und Zwischenprodukte entsprechend. Diese Restedefinitionen können untereinander, also auch zwischen den jeweiligen Vorzugsbereichen, beliebig kombiniert werden.

Erfindungsgemäß bevorzugt werden die Verbindungen der allgemeinen Formel (I), in welchen eine Kombination der vorstehend als bevorzugt (vorzugsweise) aufgeführten Bedeutungen vorliegt.

In den oben und nachstehend aufgeführten Restedefinitionen sind Kohlenwasserstoffreste, wie Alkyl oder Alkenyl - auch in Verbindungen mit Heteroatomen wie Alkoxy oder Alylthio - soweit möglich jeweils geradkettig oder verzweigt.

Bevorzugte erfindungsgemäße Verbindungen sind Stoffe der Formeln (Ia) bis (Id): 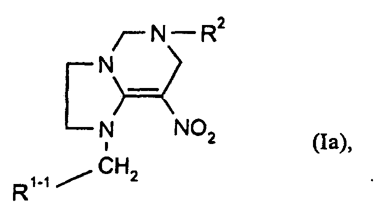 in welcher
- R¹⁻¹: für gegebenenfalls substituiertes Pyridyl steht und
- R²: die in der Erfindungsdefinition genannten Bedeutungen hat. 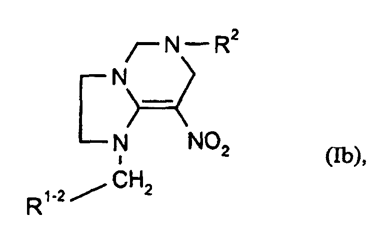
in welcher
- R¹⁻²: für gegebenenfalls substituiertes Thiazolyl steht und
- R²: die in der Erfindungsdefinition genannten Bedeutungen hat. 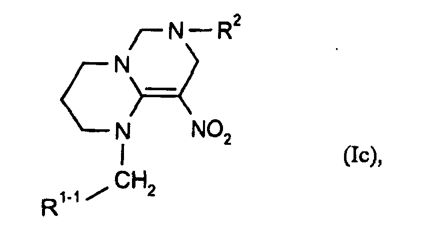
in welcher
- R¹⁻¹: für gegebenenfalls substituiertes Pyridyl steht und
- R²: die in der Erfindungsdefinition genannten Bedeutungen hat. 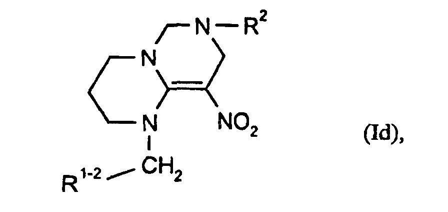
in welcher
- R¹⁻²: für gegebenenfalls substituiertes Thiazolyl steht und
- R²: die in der Erfindungsdefinition genannten Bedeutungen hat.

Bevorzugte erfindungsgemäße Verbindungen sind auch Stoffgruppen der Formeln (Ia-1), (Ib-1), (Ic-1) und (Id-1): 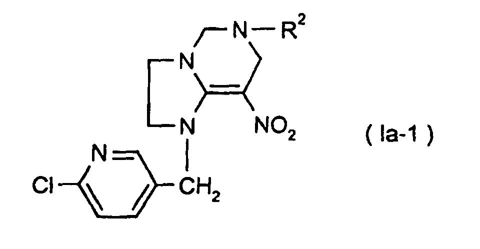 in welcher
- R²: für die oben genannten allgemeinen, bevorzugten und besonders bevorzugten Bedeutungen steht. 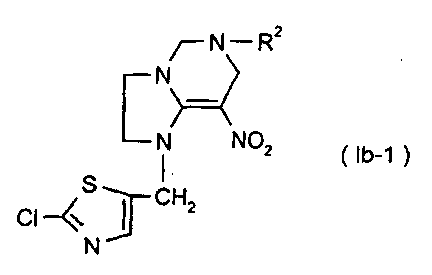 in welcher
- R²: für die oben genannten allgemeinen, bevorzugten und besonders bevorzugten Bedeutungen steht. 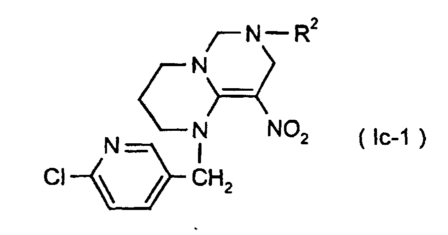 in welcher
- R²: für die oben genannten allgemeinen, bevorzugten und besonders bevorzugten Bedeutungen steht. 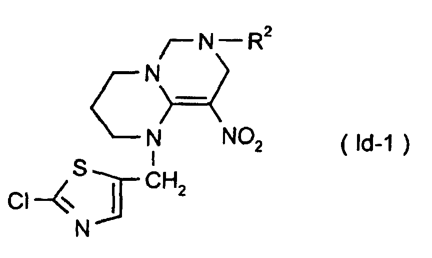 in welcher
- R²: für die oben genannten allgemeinen, bevorzugten und besonders bevorzugten Bedeutungen steht.

Beispiele für die neuen erfindungsgemäßen Verbindungen neben den Herstellungsbeispielen sind in den Tabellen a bis d aufgeführt:

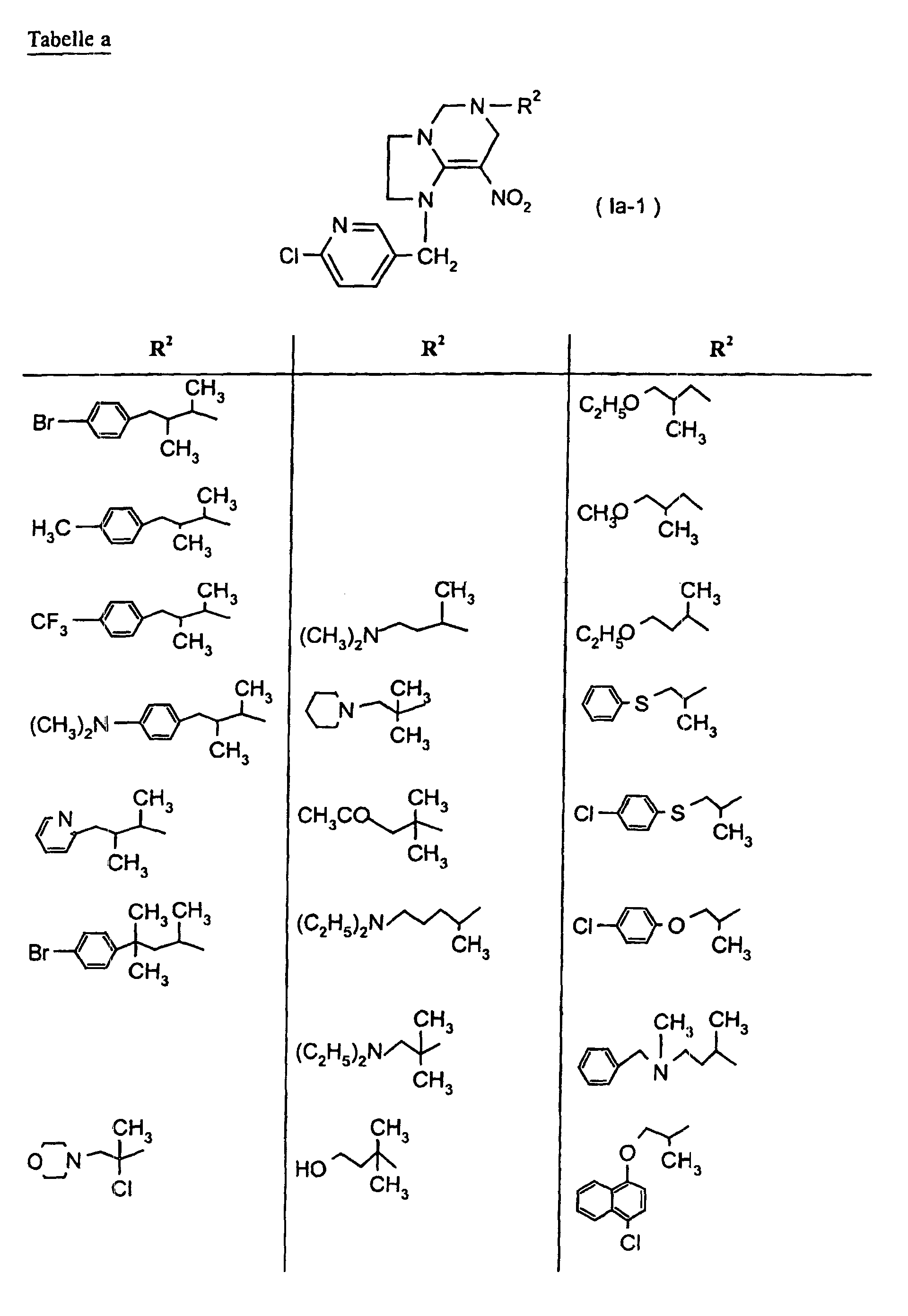

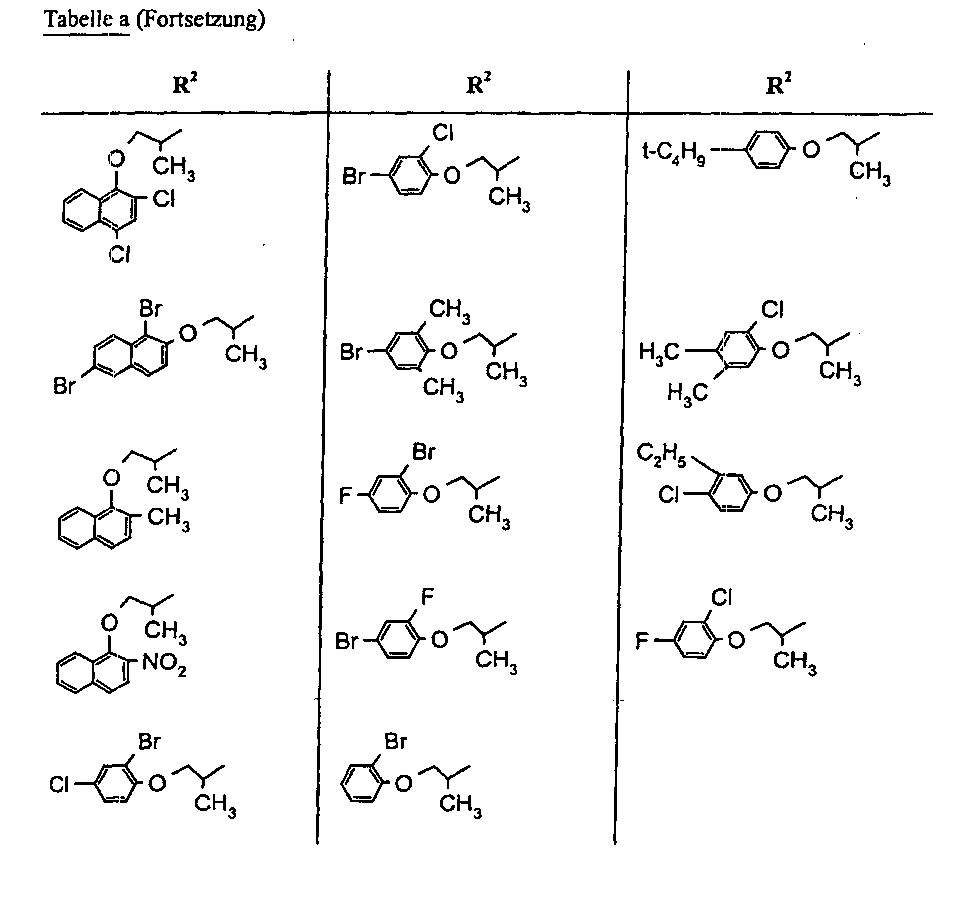

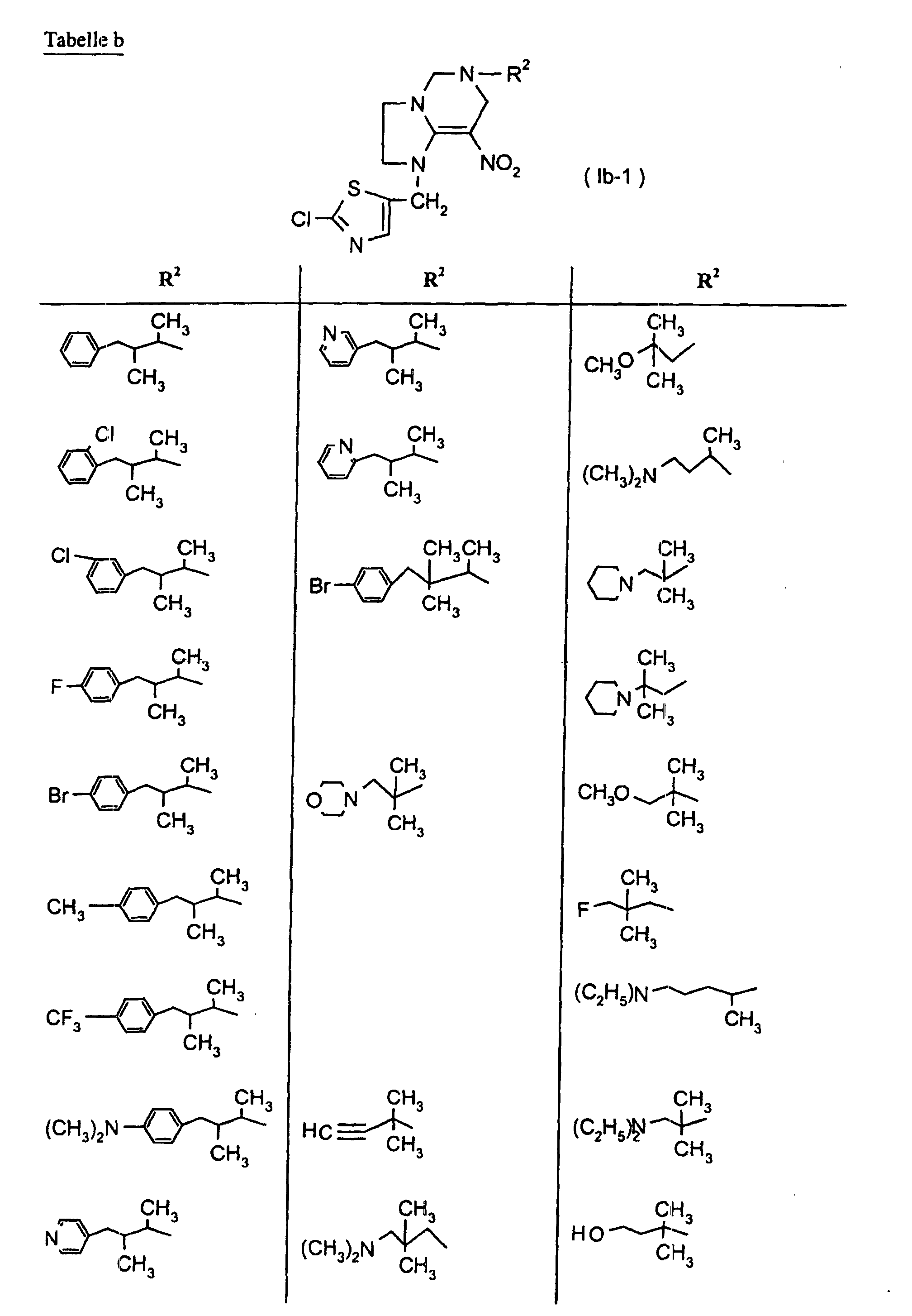

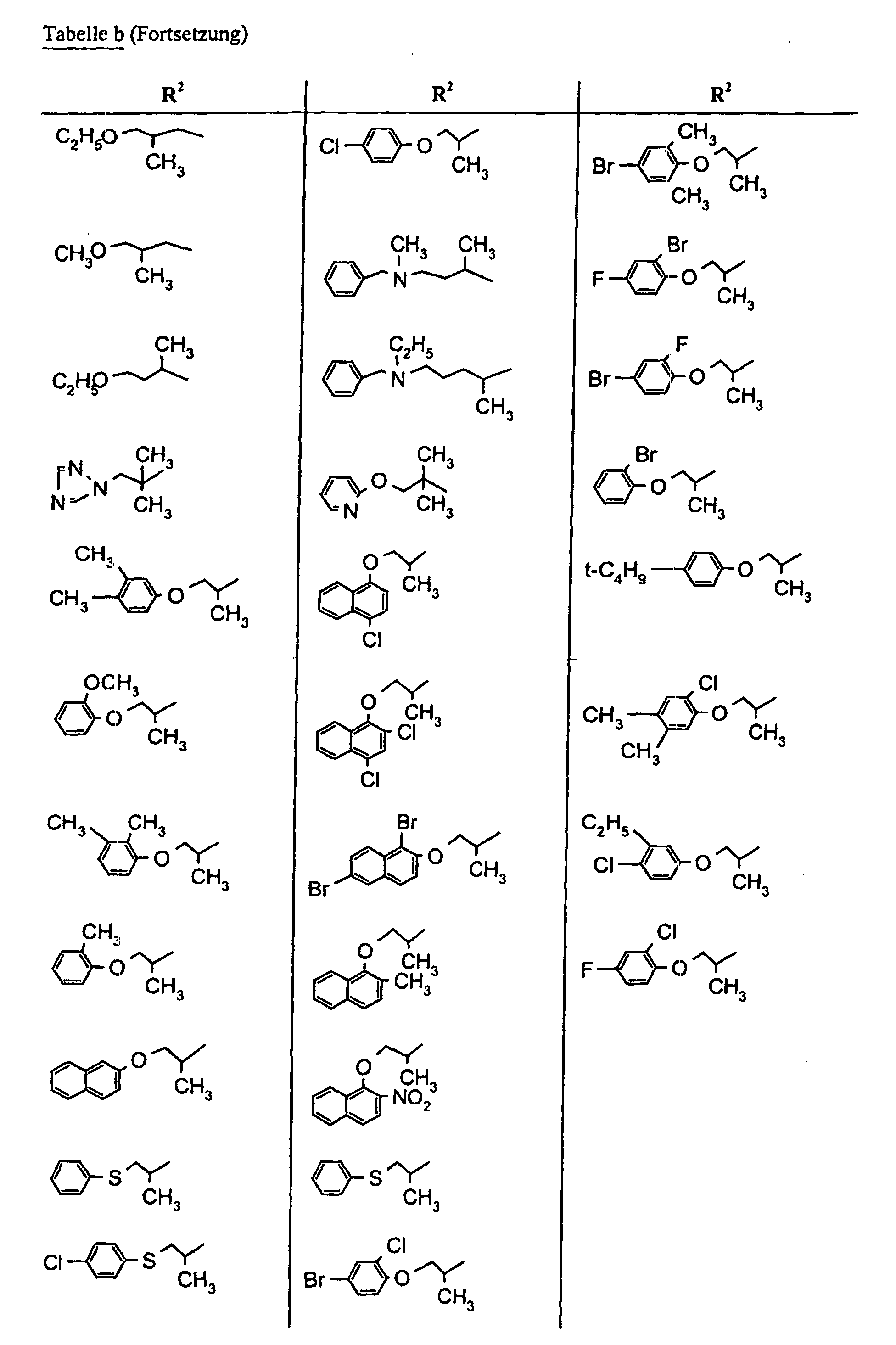

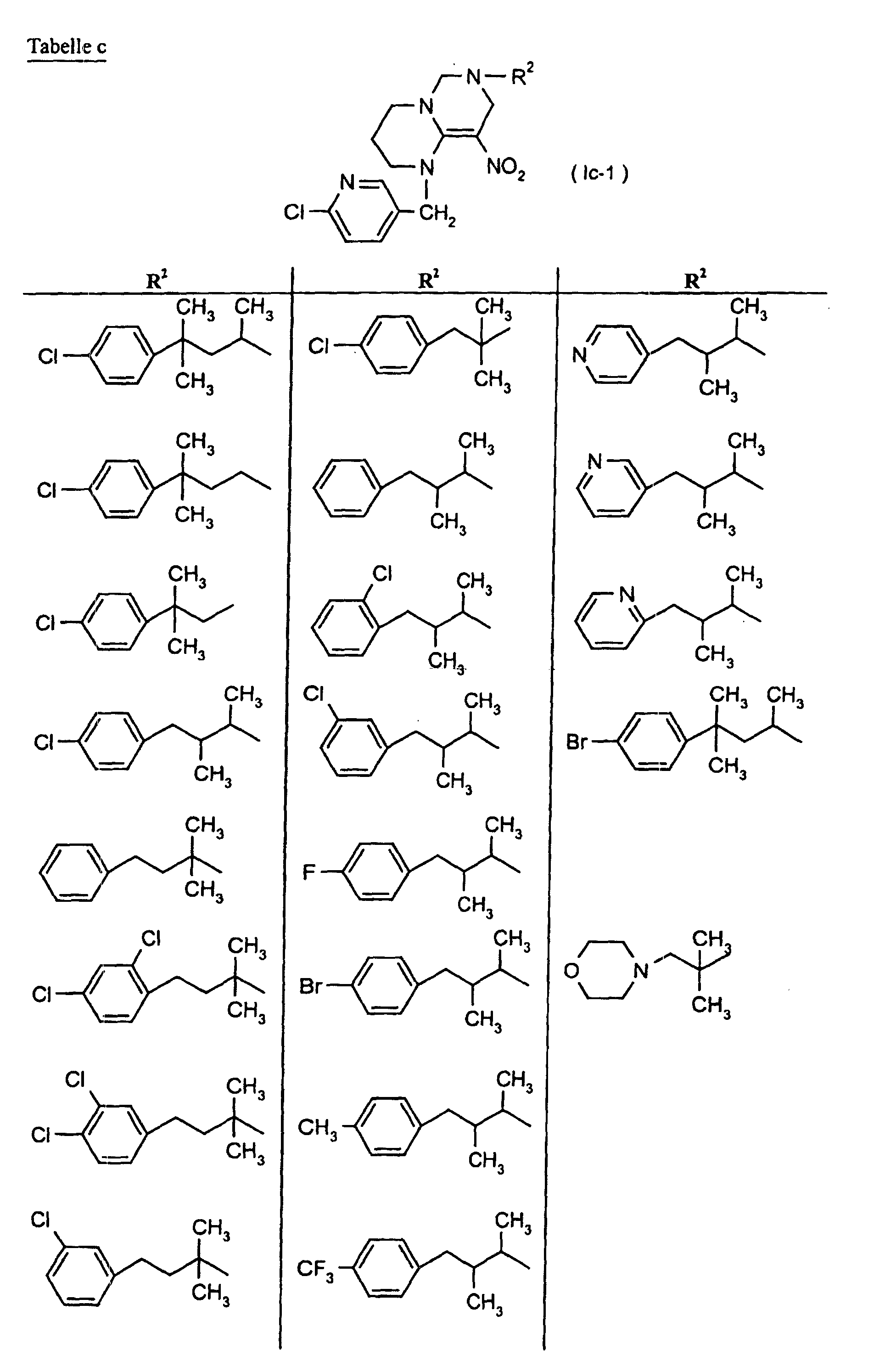

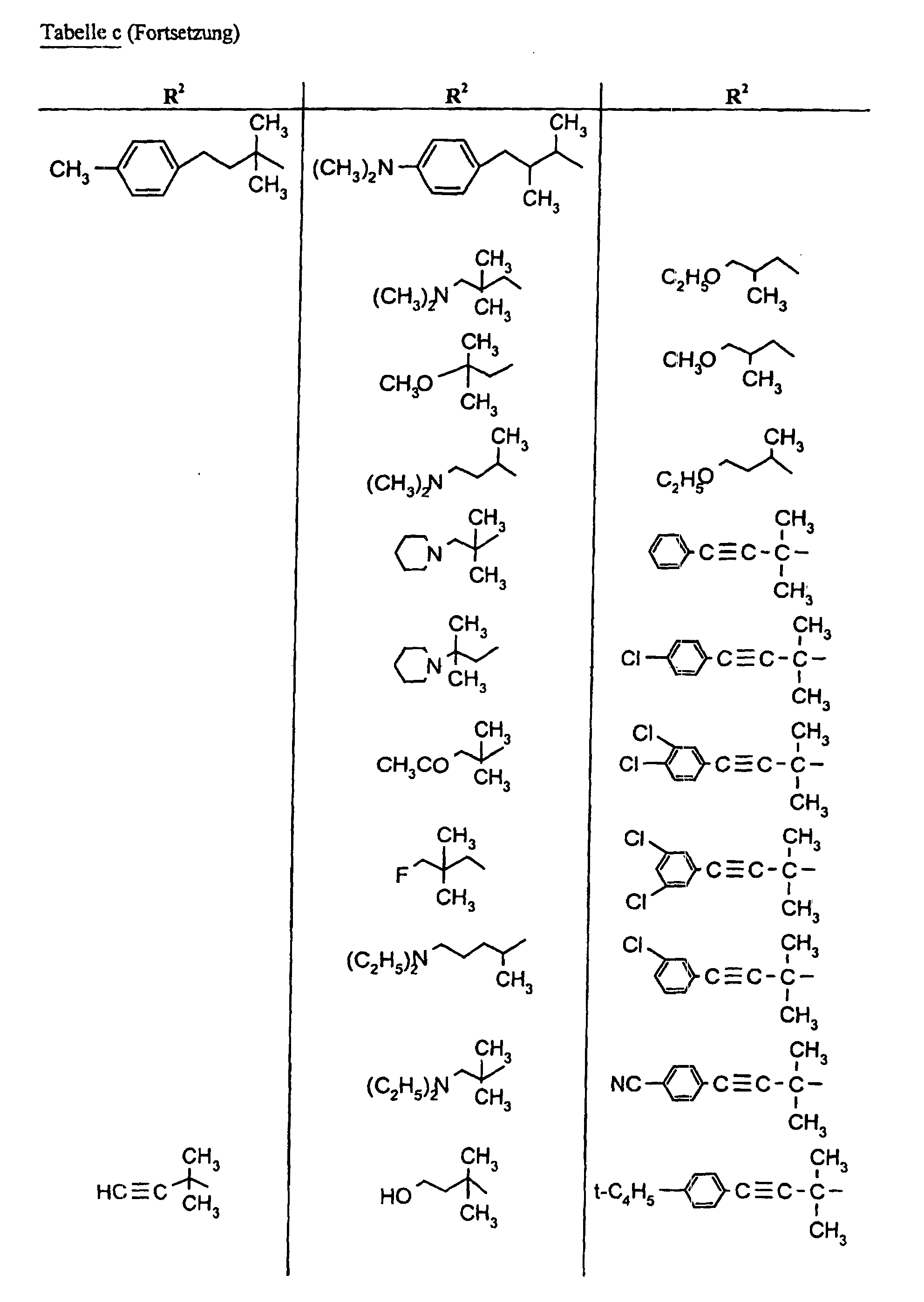

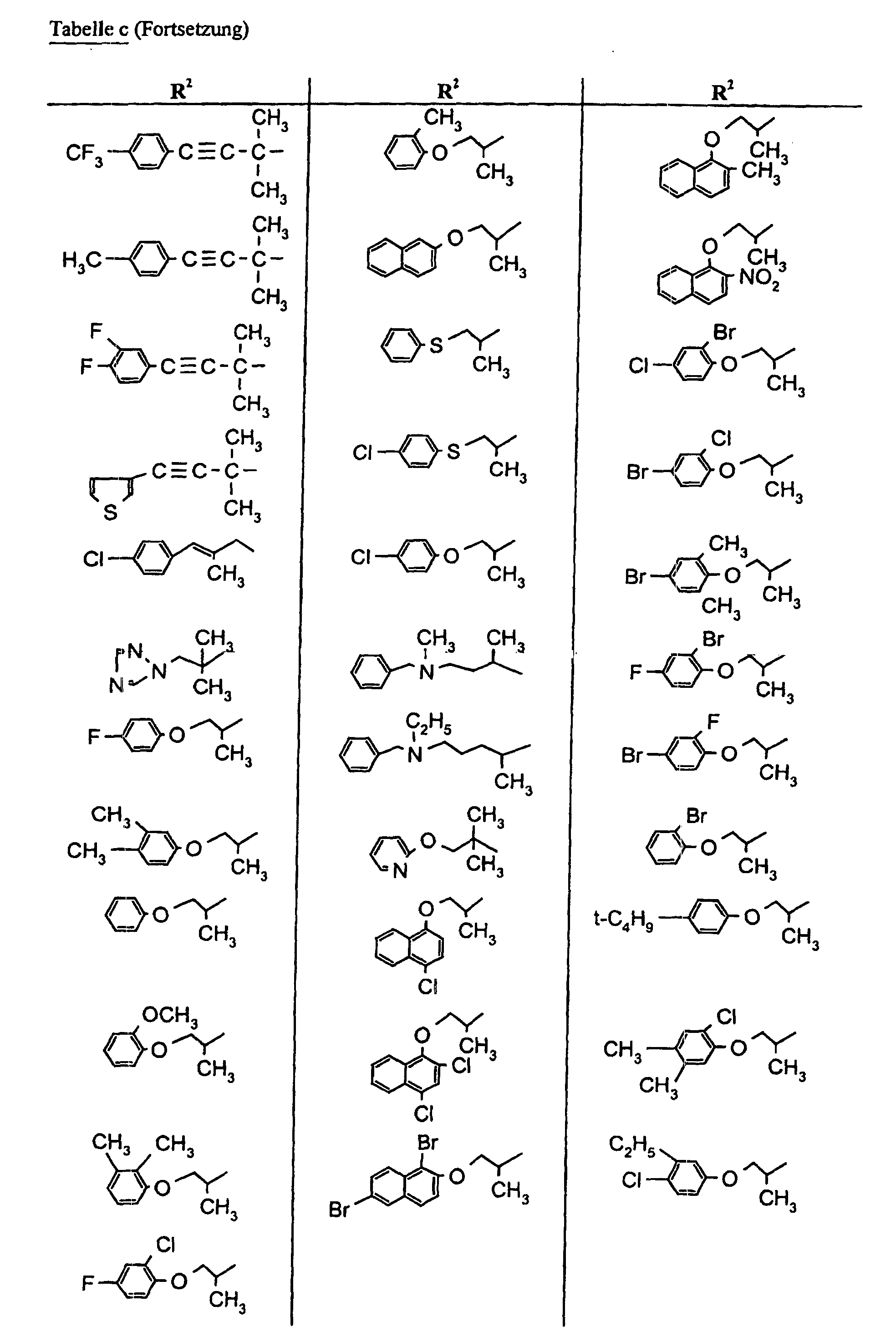

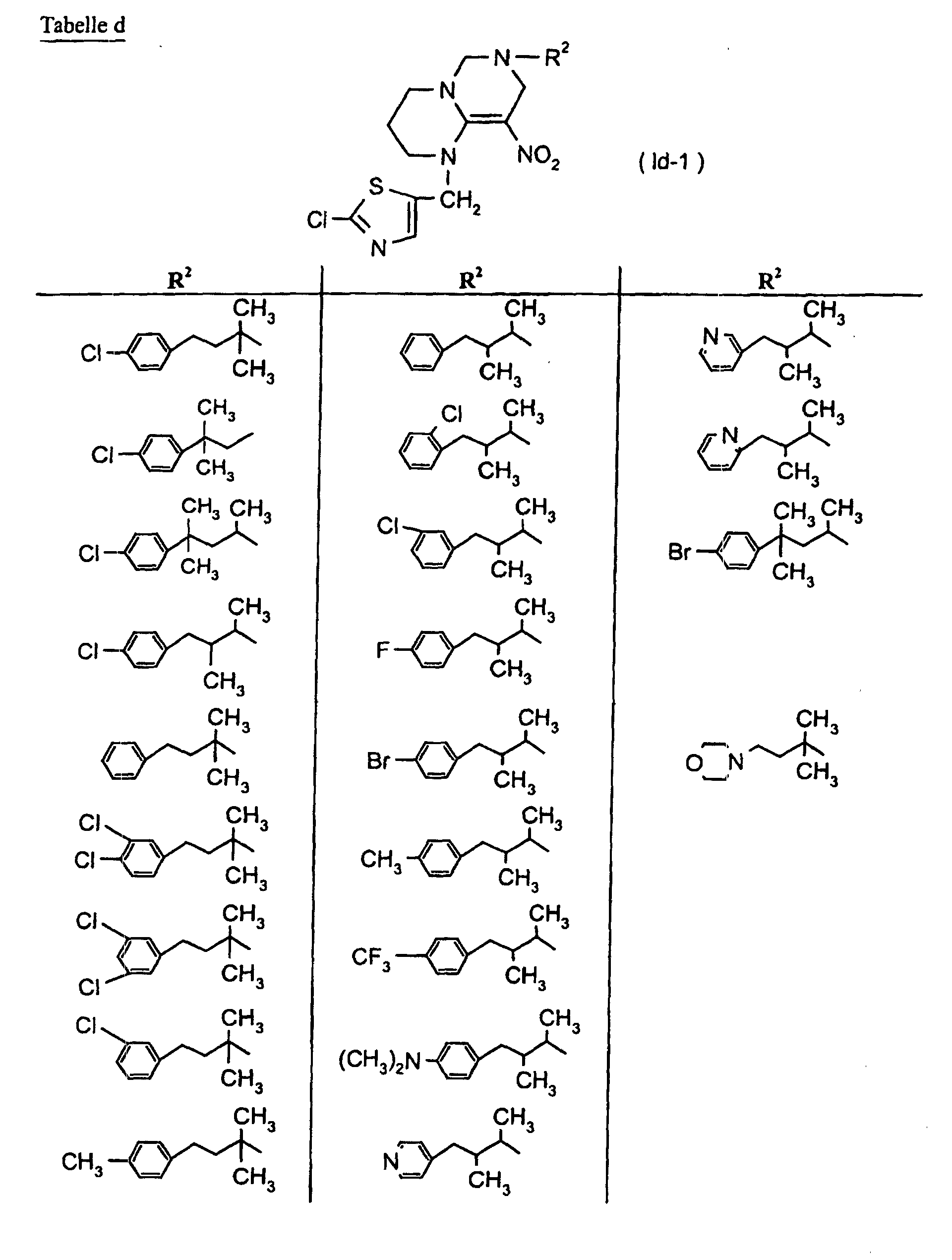

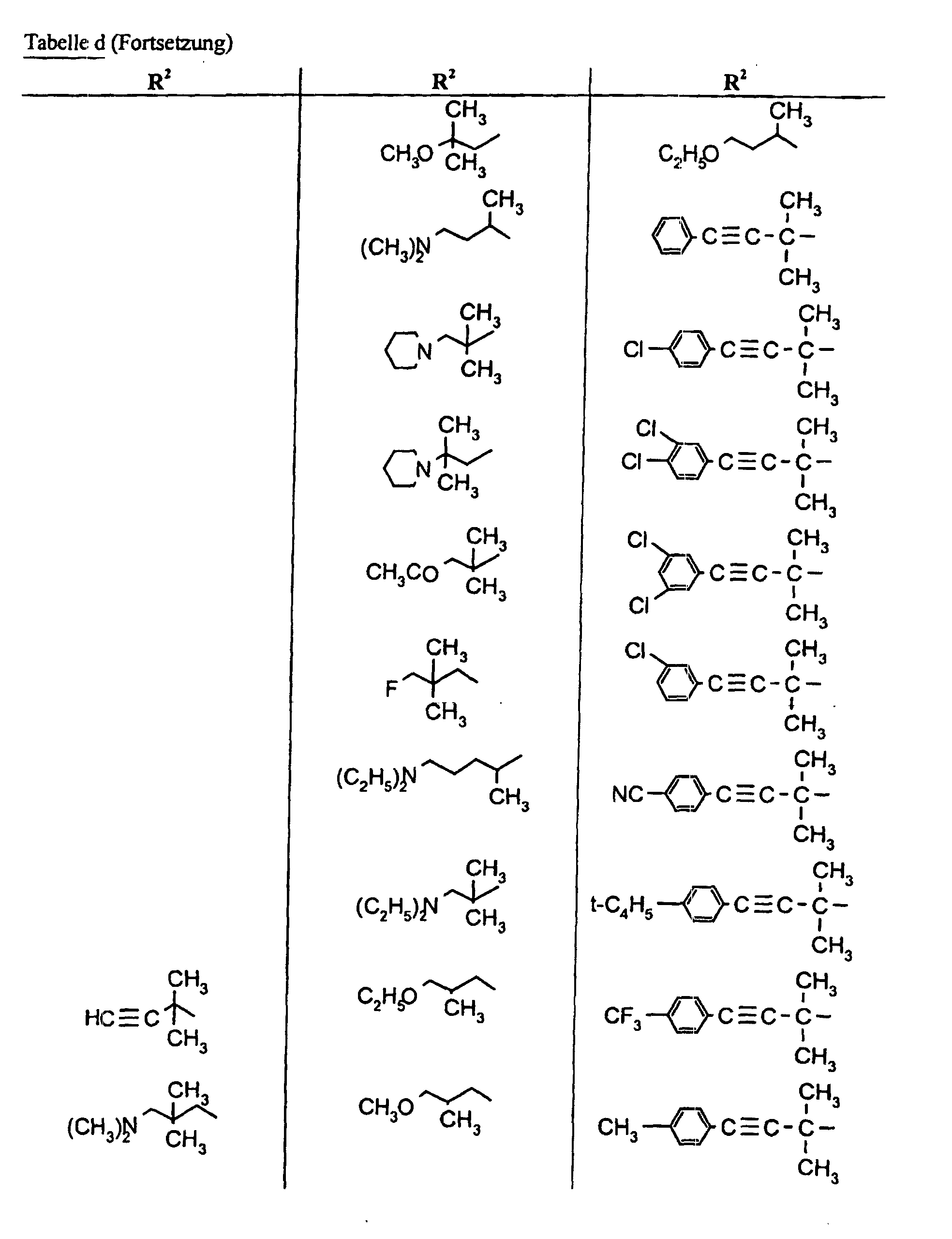

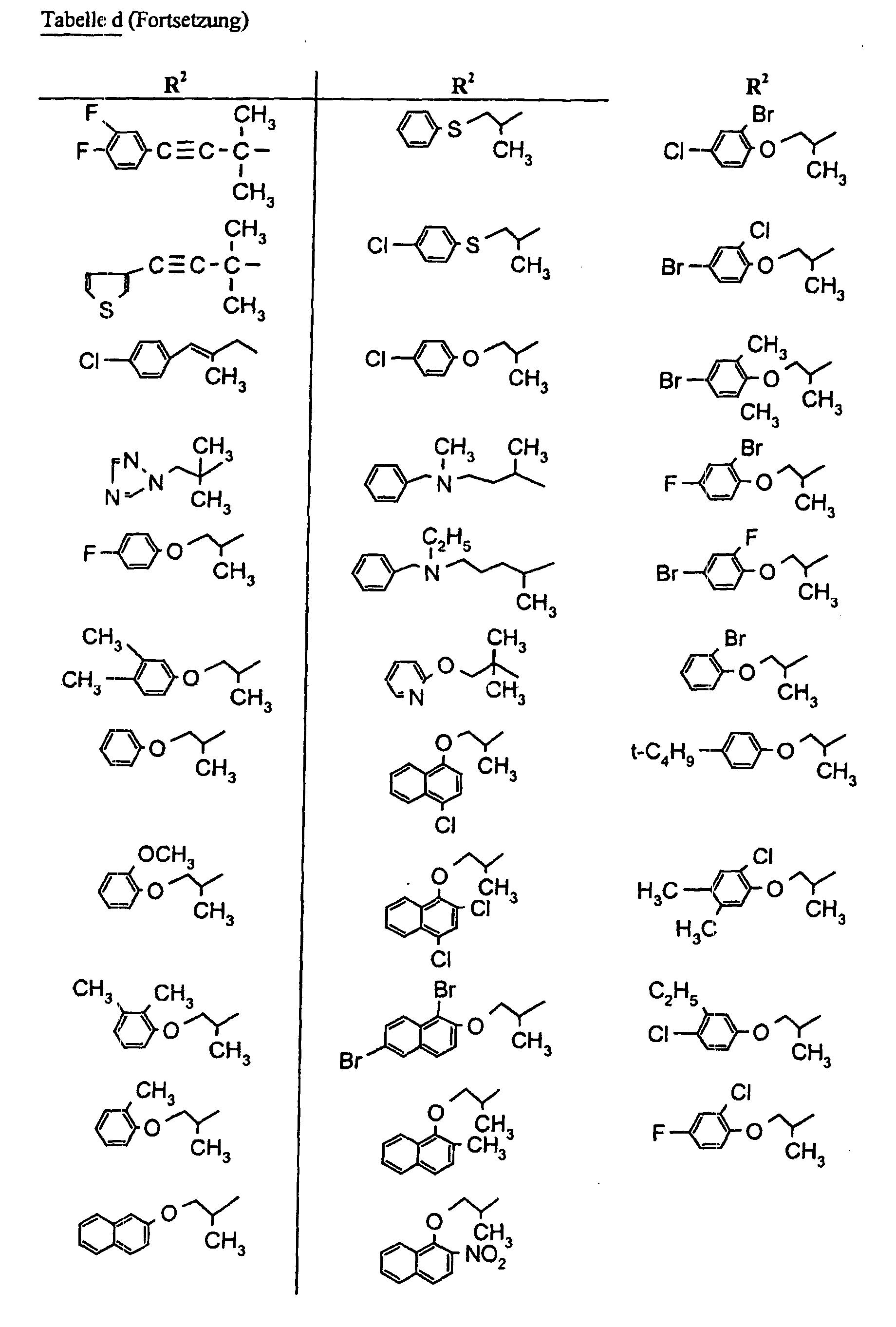

Verwendet man zur Durchführung des erfindungsgemäßen Verfahrens beispielsweise 3-(2-Chlorpyridin-5-yl-methyl)-2-nitromethylen-imidazolidin, 2-Amino-4-methyl-4(4-chlorphenyl)-pentan und 2 Mol Formaldehyd, so kann der Reaktionsablauf durch das folgende Formelschema skizziert werden:

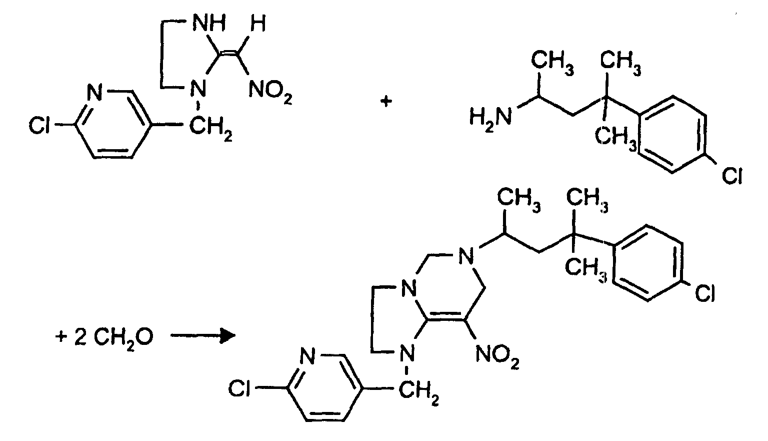

Die beim erfindungsgemäßen Verfahren als Ausgangsstoffe zu verwendenden Nitromethylen-Derivate sind durch die Formel (II) allgemein definiert. In der Formel (II) haben R¹ und n vorzugsweise bzw. besonders bevorzugt diejenigen Bedeutungen, die bereits oben im Zusammenhang mit der Beschreibung der erfindungsgemäßen Verbindungen der Formel (I) vorzugsweise bzw. besonders bevorzugt für R' und n angegeben wurden.

Die Nitromethylen-Derivate der Formel (II) sind bekannt und/oder lassen sich nach bekannten Methoden herstellen (vgl. z.B. DE-OS 2 514 402, EP-OS 136 636, EP-OS 154 178 und EP-OS 163 855).

Die beim erfindungsgemäßen Verfahren außerdem als Ausgangsstoffe zu verwendenden Amine sind durch die Formel (III) allgemein definiert. In der Formel (III) hat R² vorzugsweise bzw. besonders bevorzugt diejenige Bedeutung, die bereits oben im Zusammenhang mit der Beschreibung der erfindungsgemäßen Verbindungen der Formel (I) vorzugsweise bzw. besonders bevorzugt für R² angegeben wurde.

Die Amine der Formel (III) sind allgemein bekannte Verbindungen der organischen Chemie.

Es ist auch möglich, die Verbindungen der Formel (I) in Additionsprodukte mit Säuren zu überführen.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Schwefelsäure, Salpetersäure, Essigsäure, Oxalsäure, Malonsäure, Bernsteinsäure, Äpfelsäure, Weinsäure, Maleinsäure, Fumarsäure, Methansulfonsäure, Benzoesäure, substituierte Benzoesäuren, Ameisensäure, Chloressigsäure, Toluolfulfonsäure, Benzolsulfonsäure, Trichloressigsäure, Phthalsäure, Naphthalinsulfonsäure, Nikotinsäure, Zitronensäure und Ascorbinsäure.

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei Wasser und für die Umsetzung inerte organische Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligoin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole, wie Methanol, Ethanol, n-Propanol und Isopropanol. Bevorzugt werden Gemische aus Alkoholen und Wasser eingesezt.

Das erfindungsgemäße Verfahren wird gegebenenfalls in Gegenwart von sauren, nicht oxidierenden Katalysatoren durchgeführt. Besonders bewährt haben sich Halogenwasserstoff säuren wie Salzsäure und Bromwasserstoffsäure, Phosphorsäure, niedere Carbonsäuren wie Essigsäure und Propionsäure.

Die Reaktionstemperaturen können bei dem erfindungsgemäßen Verfahren in einem größeren Bereich variiert werden. Im allgemeinen arbeitet man bei Temperaturen zweischen -20°C und + 120°C, vorzugsweise bei Temperaturen zwischen 0°C und + 80°C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens setzt man auf 1 Mol Nitromethylen-Derivat der Formel (II), 1 bis 1,5 Mol, vorzugsweise 1 bis 1,2 Mol Amin der Formel (III) und 2 bis 4 Mol, vorzugsweise 2 bis 3 Mol Formaldehyd ein.

Die Amine der Formel (III) können gegebenenfalls als wäßrige Lösungen eingesetzt werden. Bei der Verwendung von gasförmigen Aminen der Formel (III) können diese Verbindungen durch das Gemisch aus Verdünnungsmittel, Verbindungen der Formel (II) und Formaldehyd geleitet werden. Für das erfindungsgemäße Verfahren wird Formaldehyd in wäßriger Lösung eingestzt. Die Reaktionen werden im allgemeinen in einem geeigneten Verdünnungsmittel durchgeführt und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Die Säureadditions-Salze der Verbindungen der Formel (I) können in einfacher Weise nach üblichen Salzbildungsmethoden, z.B. durch Lösung einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Pediculus humanus corporis, Haematopinus spp., Linognathus spp.

Aus der Ordnung der Mallophaga z.B. Trichodectes spp., Damalinea spp. Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Spodoptera litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp..

Die erfindungsgemäßen Verbindungen der Formel (I) zeichnen sich insbesondere durch eine hohe insektizide und akarizide Wirksamkeit aus.

Sie lassen sich mit besonders gutem Erfolg zur Bekämpfung von pflanzenschädigenden Insekten, wie beispielsweise gegen die Meerrettichblattkäfer-Larven (Phaedon cochlaeriae), die Raupen des Eulenfalters (Spodoptera frugiperda oder exigua), den Baumwollkapselwurm (Heliothis armigera), die grüne Reiszikade (Nephotettix cincticeps) und die Pfirsichblattlaus (Mycus persicae) oder zur Bekämpfung von pflanzenschädigenden Milben, wie beispielsweise gegen die gemeine Spinnmilbe oder die Bohnenspinnmilbe (Tetranychus urticae) einsetzen.

Sie zeigen darüber hinaus gute bodeninsektizide Wirkung, wie z.B. gegen Diabrotica balteata-Larven sowie gute wurzelsystemische Wirkung, wie z.B. gegen Aphis fabae.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-DichloroN-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino[alpha-(o-tolyloxy)-o-tolyl]acetat; 2-Phenylphenol (OPP), Aldimorph, Ampropylfos, Anilazin, Azaconazol,

Benalaxyl, Benodanil, Benomyl, Binapacryl, Biphenyl, Bitertanol, Blasticidin-S, Bromuconazole, Bupirimate, Buthiobate,

Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,

Dichlorophen, Diclobutrazol, Diclofluanid, Diclomezin, Dicloran, Diethofencarb, Difenoconazol, Dimethicimol, Dimethomorph, Diniconazol, Dinocap, Diphenylamin, Dipyrithion, Ditalimfos, Dithianon, Dodine, Drazoxolon,

Edifenphos, Epoxyconazole, Ethirimol, Etridiazol,

Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,

Guazatine,

Hexachlorobenzol, Hexaconazol, Hymexazol,

Imazalil, Imibenconazol, Iminoctadin, Iprobenfos (IBP), Iprodion, Isoprothiolan,

Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,

Mancopper, Mancozeb, Maneb, Mepanipyrim, Mepronil, Metalaxyl, Metconazol, Methasulfocarb, Methfuroxam, Metiram, Metsulfovax, Myclobutanil,

Nickel-dimethyldithiocarbamat, Nitrothal-isopropyl, Nuarimol,

Ofurace, Oxadixyl, Oxamocarb, Oxycarboxin,

Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,

Quintozen (PCNB),

Schwefel und Schwefel-Zubereitungen,

Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenoil, Triazoxid, Trichlamid, Tricyclazol, Tridemorph, Triflumizol, Triforin, Triticonazol,

Validamycin A, Vinclozolin,

Zineb, Ziram

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,

Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,

Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,

Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,

Edifenphos, Emamectin, Esfenvalerat, Ethiofencarb, Ethion, Ethofenprox, Ethoprophos, Etrimphos,

Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,

HCH, Heptenophos, Hexaflumuron, Hexythiazox,

Imidacloprid, Iprobenfos, Isazophos, Isofenphos, Isoprocarb, Isoxathion, Ivermectin, Lambdacyhalothrin, Lufenuron,

Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,

Naled, NC 184, NI 25, Nitenpyram

Omethoat, Oxamyl, Oxydemethon M, Oxydeprofos,

Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,

Quinalphos,

RH 5992,

Salithion, Sebufos, Silafluofen, Sulfotep, Sulprofos,

Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,

Vamidothion, XMC, Xylylcarb, YI 5301/ 5302, Zetamethrin.

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen Schaben, wie beispielsweise Periplaneta americana sowie gegen Fliegen, wie beispielsweise Musca domestica.

Sie zeigen auch eine entwicklungshemmende Wirkung, wie z.B. gegen Fliegenlarven von Lucilia cuprina.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhahtigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus

### Hautflügler wie

Sirex juvencus, Urocerus gigas, Urocerus gigas taignus, Urocerus augur

### Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze, wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/ oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethem wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der Wo 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Die Herstellung und die Verwendung der erfindungsgemäßen Wirkstoffe gehen aus den nachfolgenden Beispielen hervor.

### Herstellungsbeispiele

### Beispiel 1

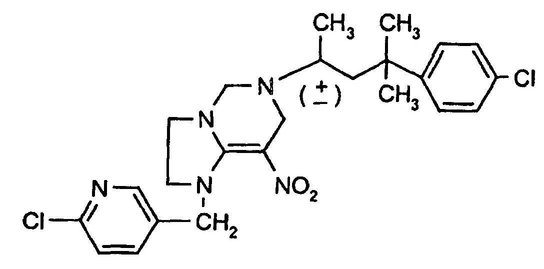

Zu einer Mischung aus 2,54 g (0,01 Mol) 3-(2-Chlorpyridin-5-yl-methyl)-2-nitromethylenimidazolidin und 2,1 g (0,01 Mol) 2-Amino-4-methyl-4(4-chlorphenyl)-pentan in 90 ml Ethanol werden bei Raumtemperatur 1,5 ml (0,02 Mol) 37 %-ige, wässrige Formaldehydlösung getropft und 3 Stunden unter Rückfluss erhitzt. Nach dem Abkühlen auf Raumtemperatur wird das Lösungsmittel im Vakuum entfernt, der Rückstand mit Ether versetzt und abgesaugt.

Man erhält 3,2 g (66 % der Theorie) 6,7-Dihydro-6-[4-methyl-4-(4-chlorphenyl)-pent-2-yl]-8-nitro-(5H)-1-(2-chlorpyridin-5-yl-methyl)-imidazolidino-[2,3-f]pyrimidin vom Schmelzpunkt 162°C.

### Beispiel 2

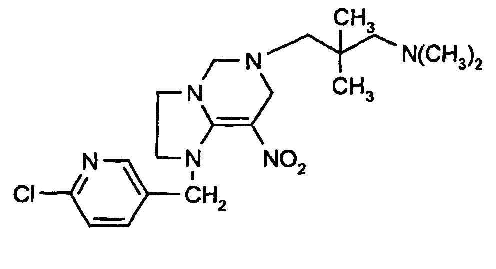

Zu einer Mischung aus 5,9 g (0,0231 Mol) 3-(2-Chlorpyridin-5-yl-methyl)-2-nitromethylenimidazolidin und 3,0 g (0,0231 Mol) 3-Dimethylamino-2,2-dimethylpropylamin in 120 ml Ethanol werden bei Raumtemperatur 4,62 ml (0,0462 Mol) 37 %-ige, wässrige Formaldehydlösung getropft und 3 Stunden unter Rückfluss erhitzt. Nach dem Abkühlen auf Raumtemperatur wird das Lösungsmittel im Vakuum entfernt, der Rückstand mit Ether verrührt und abgesaugt.

Man erhält 6,9 g (73 % der Theorie) 6,7-Dihydro-6-(3-dimethylamino-2,2-dimethyl-prop-1-yl)-8-nitro-(5H)-1-(2-chlorpyridin-5-yl-methyl)-imidazolidino-[2,3-f]-pyrimidin vom Schmelzpunkt 136°C.

### Beispiel 3

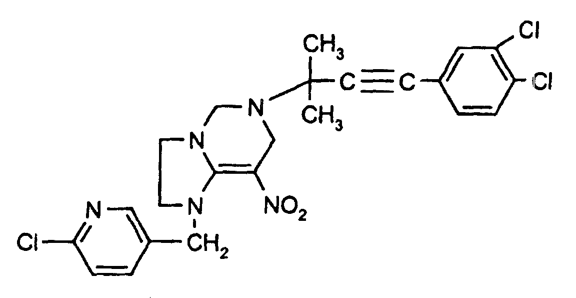

Zu einer Mischung aus 2,5 g (0,01 Mol) 3-(2-Chlor-pyridin-5-yl-methyl)-2-nitromethylenimidazolidin und 2,3 g (0,01 Mol) 2-Amino-2-methyl-4-(3,4-dichlorphenyl)-but-3-in in 90 ml Ethanol werden bei Raumtemperatur 2,0 ml (0,02 Mol) 30 %-ige, wässrige Formaldehydlösung getropft und 3 Stunden unter Rückfluss erhitzt. Nach dem Abkühlen auf Raumtemperatur wird das Lösungsmittel im Vakuum entfernt, der Rückstand mit Ether verrührt und abgesaugt.

Man erhält 3,6 g (73 % der Theorie) 6,7-Dihydro-6-[2-methyl-4-(3,4-dichlorphenyl)-but-3-in-2-yl]-8-nitro-(5H)-1-(2-chlorpyridin-5-yl-methyl)-imidazolidino-[2,3-f]-pyrimidin vom Schmelzpunkt 130°C.

Analog den Beispielen 1 bis 3 sowie gemäß den allgemeinen Angaben zur Herstellung werden die in der nachfolgenden Tabelle angegebenen Verbindungen der Formel (IA) erhalten:

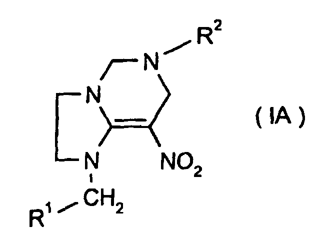

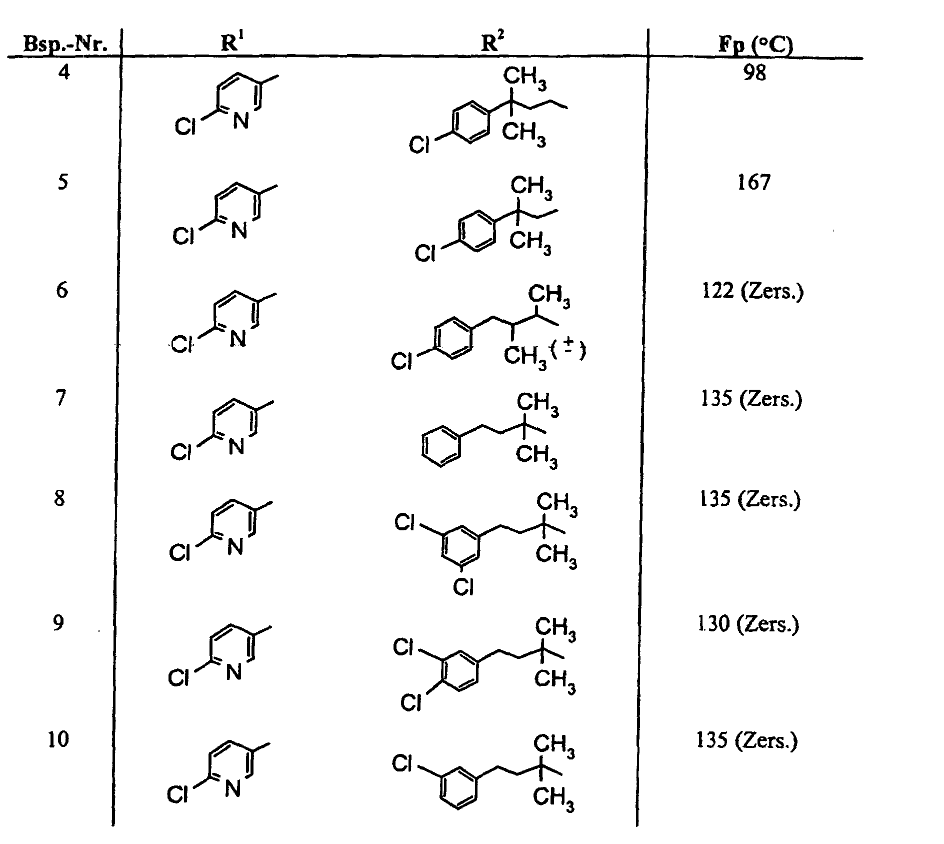

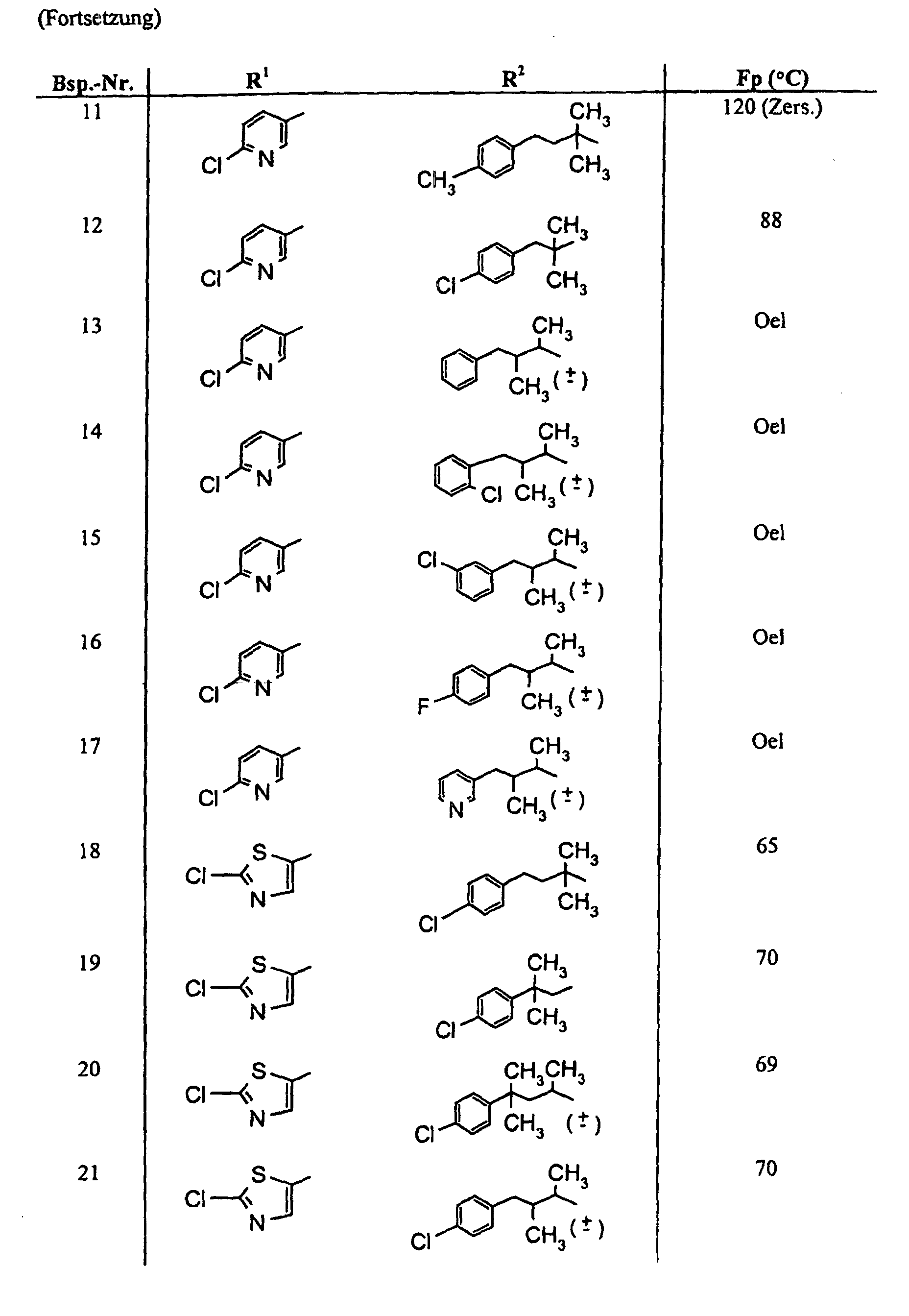

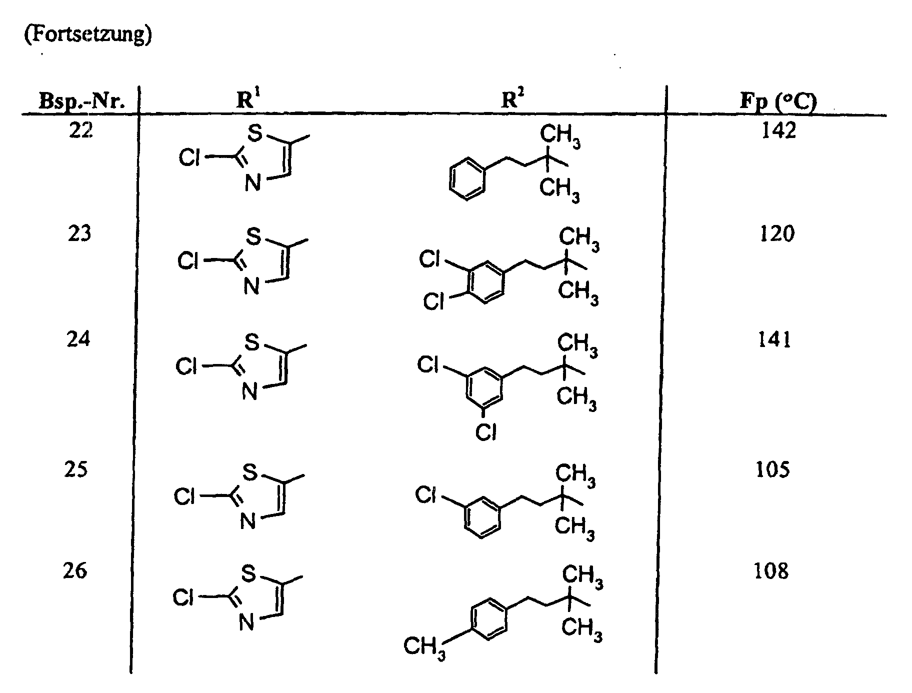

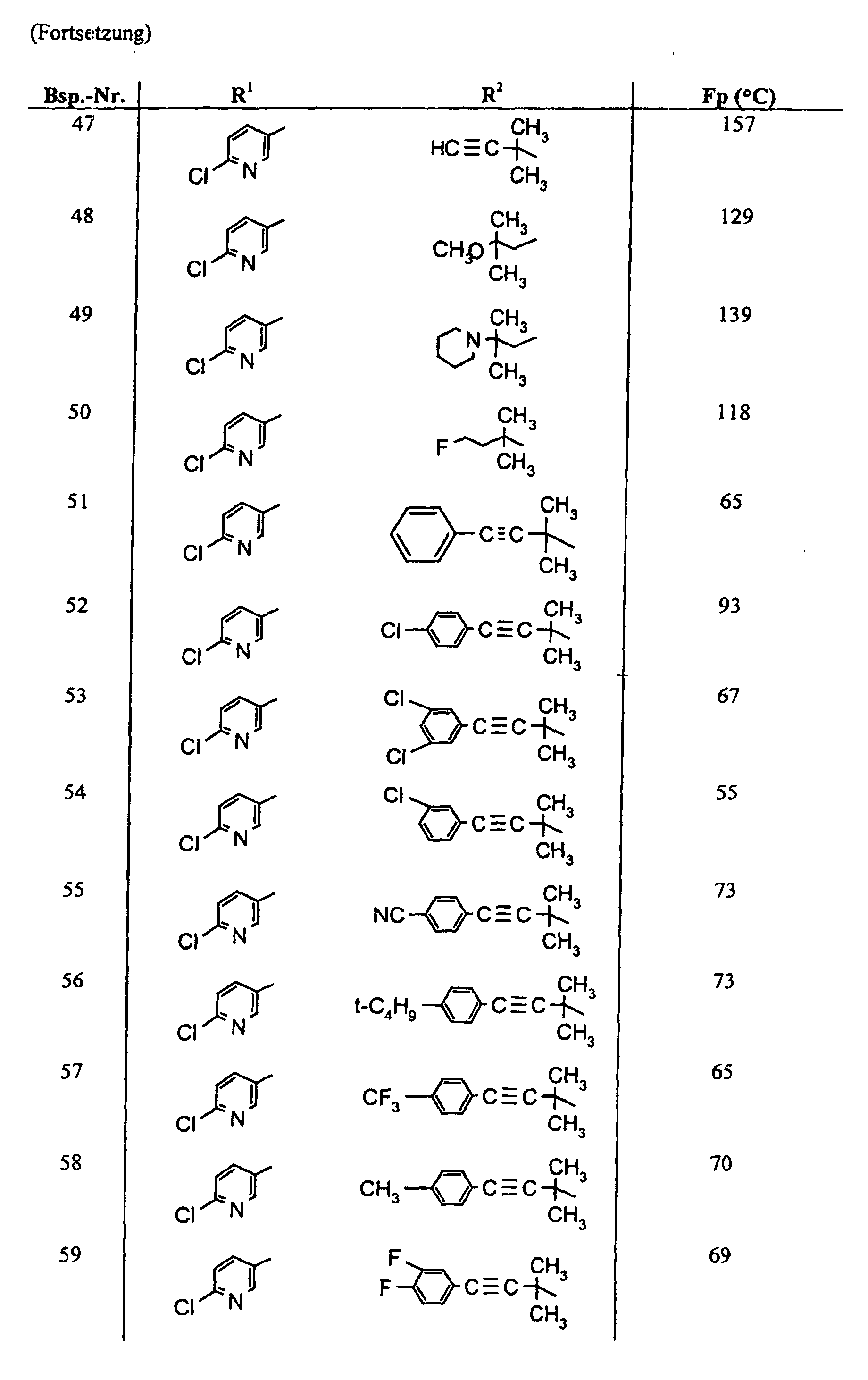

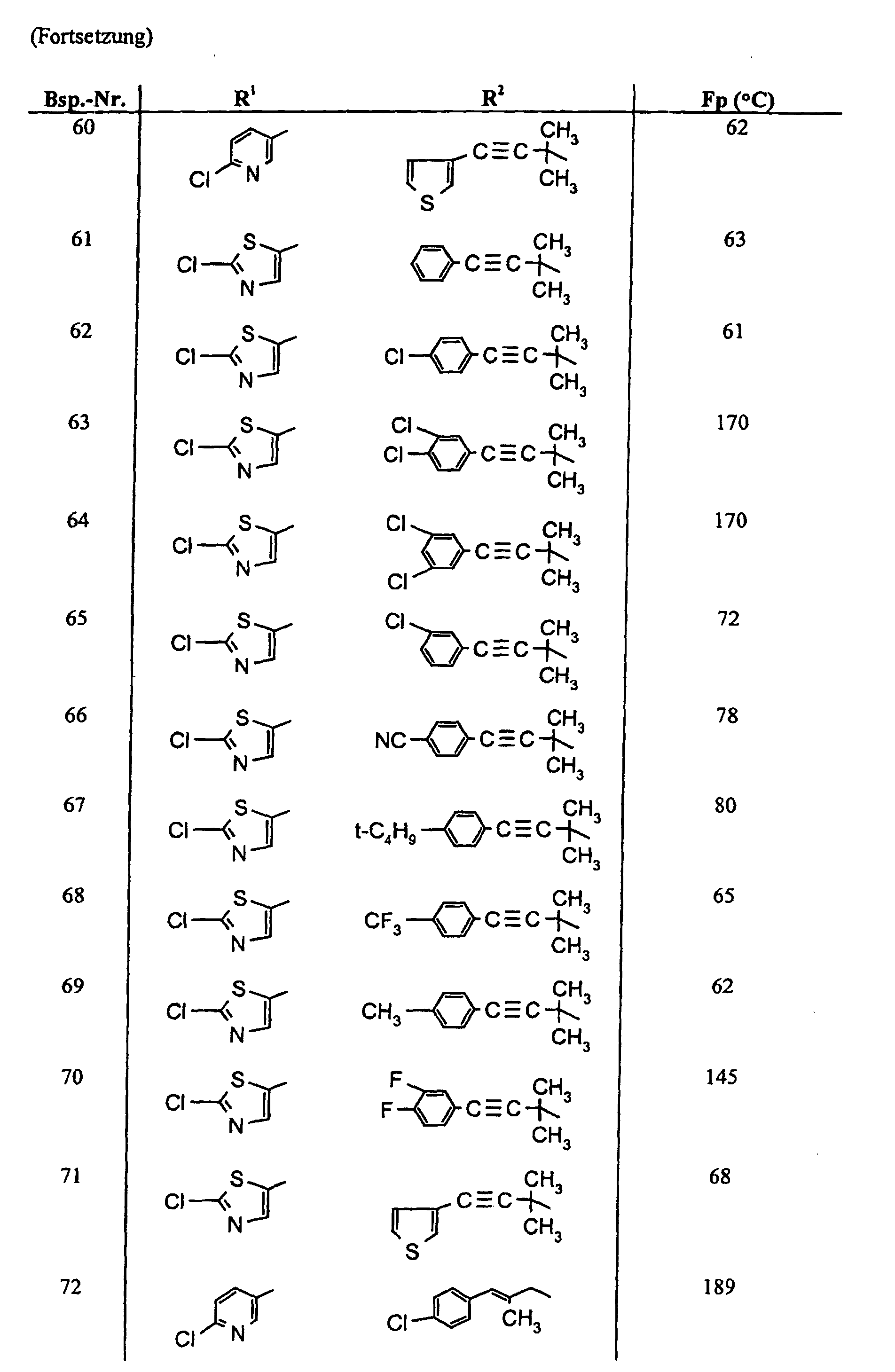

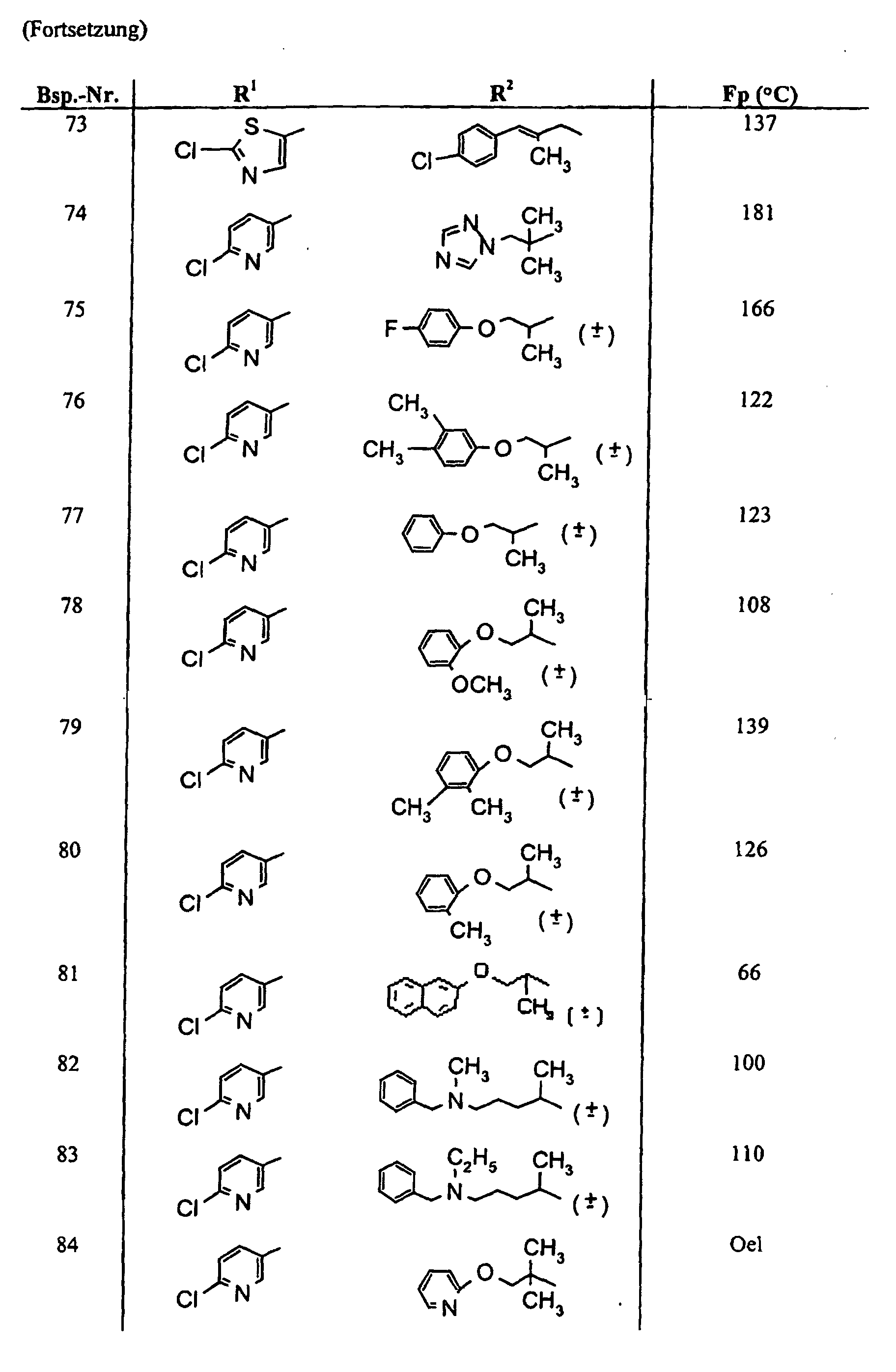

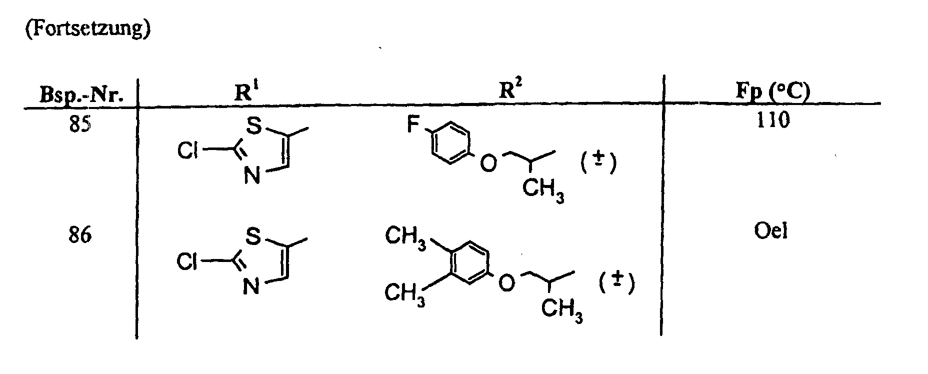

### Anwendungsbeispiele

### Beispiel A

| **Phaedon-Larven-Test** | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, dass alle Käfer-Larven abgetötet wurden; 0 % bedeutet, dass keine Käfer-Larven abgetötet wurden.

In diesem Test zeigen z.B. die Verbindungen gemäß den Herstellungsbeispielen 3, 4, 5, 6, 7, 8, 9, 10, 11, 20, 21, 24, 46, 51, 52, 53, 54, 55, 56, 57, 58, 59, 60, 67, 85 und 86 bei einer beispielhaften Wirkstoffkonzentration von 0,1 % einen Abtötungsgrad von 100 % nach 7 Tagen.

### Beispiel B

| **Plutella-Test** | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, dass alle Käfer-Larven abgetötet wurden; 0 % bedeutet, dass keine Käfer-Larven abgetötet wurden.

In diesem Test zeigen z.B. die Verbindungen gemäß den Herstellungsbeispielen 1, 4, 6, 7, 8, 10, 11, 22, 26, 51, 56, 59, 62, 66 und 86 bei einer beispielhaften Wirkstoffkonzentration von 0,1 % einen Abtötungsgrad von 100 % nach 7 Tagen.

### Beispiel C

| **Spodoptera-Test** | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100 %, dass alle Käfer-Larven abgetötet wurden; 0 % bedeutet, dass keine Käfer-Larven abgetötet wurden.

In diesem Test zeigen z.B. die Verbindungen gemäß den Herstellungsbeispielen 7, 8, 9, 10, 11, 12, 22, 23, 25, 26, 51, 55, 59 und 70 bei einer beispielhaften Wirkstoffkonzentration von 0,1 % einen Abtötungsgrad von 100 % nach 7 Tagen.

### Beispiel F

| **Nephotettix-Test** | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzenitration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, dass alle Zikaden abgetötet wurden; 0 % bedeutet, dass keine Zikaden abgetötet wurden.

In diesem Test zeigen z.B. die Verbindungen gemäß den Herstellungsbeispielen 8, 10, 11, 12, 20, 22, 23, 24, 25, 68, 70 und 71 bei einer beispielhaften Wirkstoffkonzentration von 0,1 % einen Abtötungsgrad von 100 % nach 6 Tagen.

### Beispiel G

| **Myzus-Test** | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in Prozent bestimmt. Dabei bedeutet 100 %, dass alle Blattläuse abgetötet wurden; 0 % bedeutet, dass keine Blattläuse abgetötet wurden.

In diesem Test zeigen z.B. die Verbindungen gemäß den Herstellungsbeispielen 1, 6, 8, 9, 10, 12, 22, 25, 26, 54, 59, 60 und 86 bei einer beispielhaften Wirkstoffkonzentration von 0,1 % einen Abtötungsgrad von 100% nach 6 Tagen.

### Beispiel H

| **Tetranychus-Test** (OP-resistent/Tauchbehandlung) | |
|---|---|
| Lösungsmittel | 7 Gewichtsteile |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

In diesem Test zeigen z.B. die Verbindungen gemäß den Herstellungsbeispielen 13, 14, 15, 16 und 17 bei einer beispielhaften Wirkstoffkonzentration von 0,1 % einen Abtötungsgrad von 98 % nach 13 Tagen.

### Beispiel J

| **Grenzkonzentrations-Test** / Wurzelsystemische Wirkung | |
|---|---|
| Testinsekt | Aphis fabae |
| Lösungsmittel | 4 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Bohnen (Vicia faba). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 6 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

In diesem Test zeigen z.B. die Verbindungen gemäß den Herstellungsbeispielen 1 und 6 bei einer beispielhaften Wirkstoffkonzentration von 20 ppm einen Abtötungsgrad von 100 %.

### Beispiel K

| **Blowfly-Larven-Test/**Entwicklungshemmende Wirkung | |
|---|---|
| Testtiere | Lucilia cuprina-Larven |
| Emulgator | 35 Gewichtsteile Ethylenglykolmonomethylether |
| | 35 Gewichtsteile Nonylphenolpolyglykolether |

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Emulsionskonzentrat mit Wasser auf die jeweils gewünschte Konzentration.

Etwa 20 Lucilia cuprina res.-Larven werden in ein Teströhrchen gebracht, welches ca. 1 cm³ Pferdefleisch und 0,5 ml der Wirkstoffzubereitung enthält. Nach 24 Stunden und 48 Stunden wird die Wirksamkeit der Wirkstoffzubereitung ermittelt. Die Teströhrchen werden im Becher mit Sand-bedecktem Boden überführt. Nach weiteren 2 Tagen werden die Teströhrchen entfernt und die Puppen gezählt.

Die Wirkung der Wirkstoffzubereitung wird nach der Zahl der geschlüpften Fliegen nach 1,5-facher Entwicklungsdauer einer unbehandelten Kontrolle beurteilt. Dabei bedeutet 100 %, dass keine Fliegen geschlüpft sind; 0 % bedeutet, dass alle Fliegen normal geschlüpft sind.

In diesem Test zeigen z.B. die Verbindungen gemäß den Herstellungsbeispielen 1, 3, 5, 6, 7, 10, 12, 18, 19, 20, 22, 23, 24, 25, 26, 51, 53, 55, 56, 58, 59, 60, 61, 62, 63, 64, 65, 66, 67, 69, 70, 71 und 73 bei einer beispielhaften Wirkstoffkonzentration von 1000 ppm eine Abtötung von 100 %.

### Beispiel L

| **Test mit Fliegen (Musca domestica)** | |
|---|---|
| Testtiere | adulte Musca domestica, Stamm Reichswald (OP, SP, Carbamatresistent) |
| Lösungsmittel | 35 Gewichtsteile Ethylenglykolmonomethylether |
| | 35 Gewichtsteile Nonylphenolpolyglykolether |

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierschalen (102 \f "Symbol" 9,5 cm) pipettiert, die sich in Petrischalen entsprechender Größe befinden. Nach Trocknung der Filterscheiben werden 25 Testtiere in die Petrischale überführt und abgedeckt.

Nach 1, 3, 5 und 24 Stunden wird die Wirksamkeit der Wirkstoffzubereitung ermittelt. Dabei bedeutet 100 %, dass alle Fliegen abgetötet wurden; 0 % bedeutet, dass keine Fliegen abgetötet wurden.

In diesem Test zeigen z.B. die Verbindungen gemäß den Herstellungsbeispielen 3, 6, 7, 10, 12, 18, 19, 20, 21, 22, 23, 24, 25, 26, 53, 56, 59, 60, 61, 62, 63, 64, 65, 66, 67, 69, 70, 71 und 73 bei einer beispielhaften Wirkstoffkonzentration von 1000 ppm eine Abtötung von 100 %.

### Beispiel M

| **Schabentest** | |
|---|---|
| Testtiere | Periplaneta americana |
| Lösungsmittel | 35 Gewichtsteile Ethylenglykolmonomethylether |
| | 35 Gewichtsteile Nonylphenolpolyglykolether |

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierscheiben (102 \f "Symbol" 9,5 cm) pipettiert, die sich in Petrischalen entsprechender Größe befinden. Nach Trocknung der Filterscheiben werden 5 Testtiere P. americana überführt und abgedeckt.

Nach 3 Tagen wird die Wirksamkeit der Wirkstoffzubereitung bestimmt. Dabei bedeutet 100 %, dass alle Schaben abgetötet wurden; 0 % bedeutet, dass keine Schaben abgetötet wurden.

In diesem Test zeigen z.B. die Verbindungen gemäß den Herstellungsbeispielen 1, 5, 10 und 23 bei einer beispielhaften Wirkstoffkonzentration von 1000 ppm eine Abtötung von 100 %.

## Patentansprüche

1. Substituierte Tetrahydro-5-nitro-pyrimidine der Formel (I) 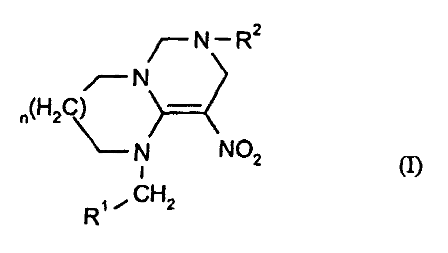 in welcher
n für 0 oder 1 steht,
R¹ für gegebenenfalls einfach bis dreifach, gleich oder verschieden substituiertes Pyridyl oder für gegebenenfalls einfach bis zweifach, gleich oder verschieden substituiertes Thiazolyl steht, wobei jeweils als Substituenten in Frage kommen:
Halogen, Cyano, Nitro, C₁-C₄-Alkyl, C₁-C₄-Alkoxy, C₁-C₄-Alkylthio, C₁-C₄-Halogenalkyl, C₁-C₄-Halogenalkoxy und C₁-C₄-Halogenalkylthio,
R² für eine der folgenden Gruppierungen steht: 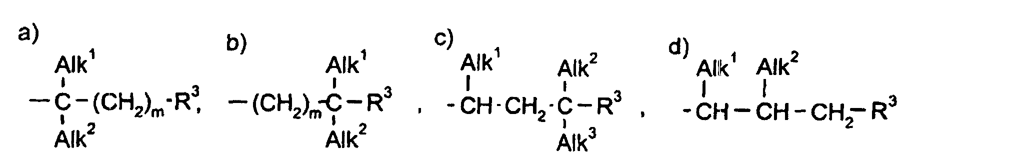 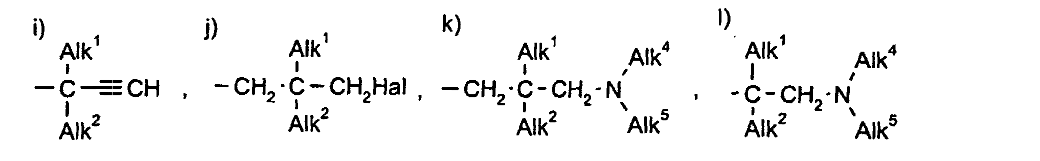 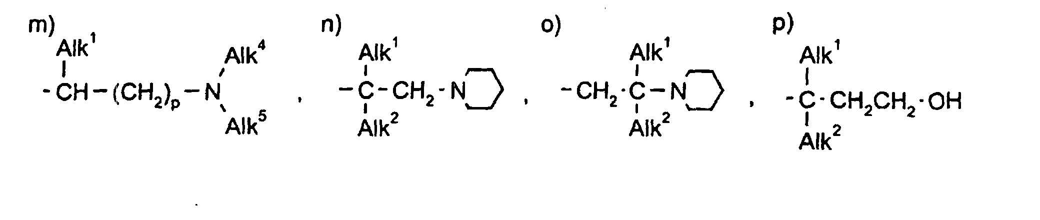 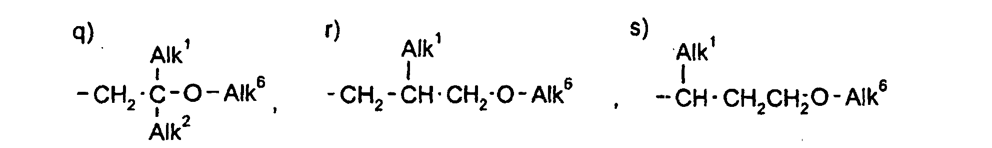 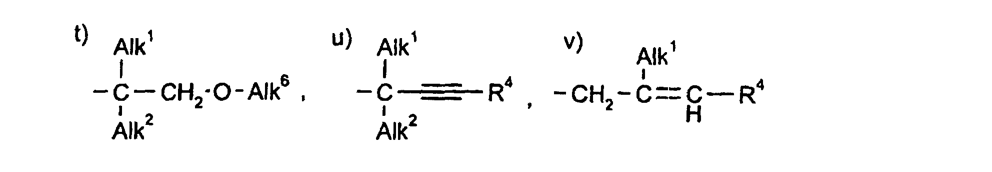 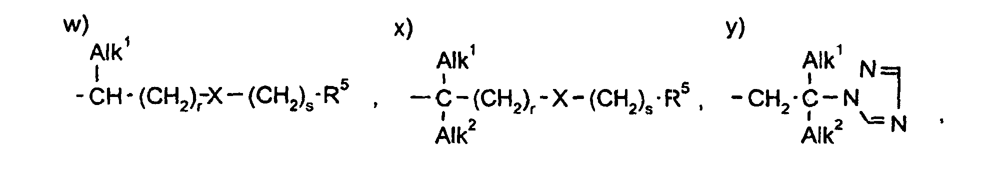
wobei
Alk¹ bis Alk⁶ unabhängig voneinander für C₁-C₄-Alkyl stehen,
Hal für Fluor, Chlor oder Brom steht,
m für 1 oder 2 steht,
p für 1, 2 oder 3 steht,
r und s unabhängig voneinander für 0, 1, 2, 3 oder 4 stehen,
R³ für gegebenenfalls einfach bis fünffach, gleich oder verschieden substituiertes Phenyl oder für gegebenenfalls einfach bis vierfach, gleich oder verschieden substituiertes Pyridyl steht, wobei jeweils als Substituenten in Frage kommen:
Halogen, Cyano, Nitro, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Amino, C₁-C₄-Alkylamino und Di(C₁-C₄-Alkyl)amino;
sowie für gegebenenfalls einfach bis zweifach, gleich oder verschieden substituiertes N-Morpholino, wobei als Substituenten in Frage kommen:
Halogen, C₁-C₄-Alkyl, C₁-C₄-Alkoxy und C₁-C₄-Halogenalkyl,
R⁴ für gegebenenfalls einfach bis fünffach, gleich oder verschieden substituiertes Phenyl oder für gegebenenfalls einfach bis dreifach, gleich oder verschieden substituiertes Thienyl steht, wobei jeweils als Substituenten in Frage kommen:
Halogen, Cyano, Nitro, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Amino, C₁-C₄-Alkylamino und Di(C₁-C₄-Alkyl)amino,
R⁵ für gegebenenfalls einfach bis fünffach, gleich oder verschieden substituiertes Phenyl, gegebenenfalls einfach bis dreifach, gleich oder verschieden substituiertes Naphthyl oder für gegebenenfalls einfach bis vierfach, gleich oder verschieden substituiertes Pyridyl steht, wobei jeweils als Substituenten in Frage kommen:
Halogen, Cyano, Nitro, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, Amino, C₁-C₄-Alkylamino und Di(C₁-C₄-Alkyl)amino und
X für Sauerstoff, Schwefel oder die Gruppierung NAlk⁷ steht, wobei
Alk⁷ für C₁-C₄-Alkyl steht.

2. Verbindungen der Formel (I) gemäß Anspruch 1, in welcher
n für 0 oder 1 steht,
R¹ für gegebenenfalls einfach bis zweifach, gleich oder verschieden substituiertes Pyridyl oder für gegebenenfalls einfach substituiertes Thiazolyl steht, wobei jeweils als Substituenten in Frage kommen:
Fluor, Chlor, Brom, Cyano, Nitro, Methyl, Ethyl, Methoxy, Methylthio, Trifluormethyl, Trifluormethoxy und Trifluormethylthio.
R² für eine der folgenden Gruppierungen steht: 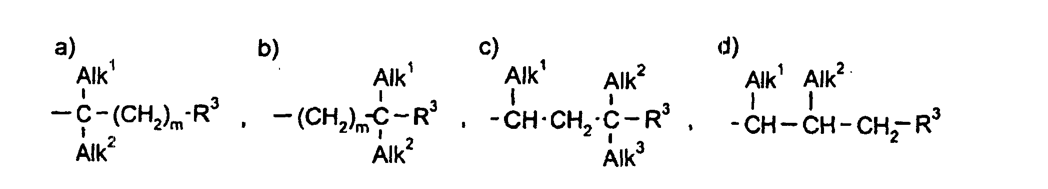 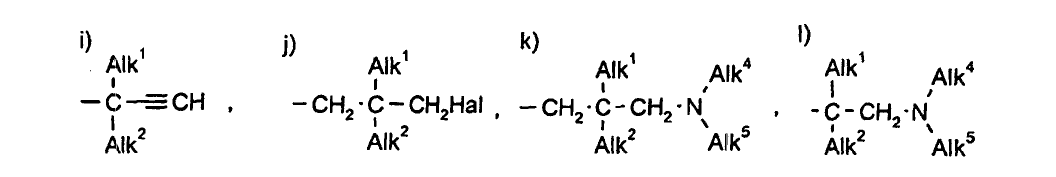 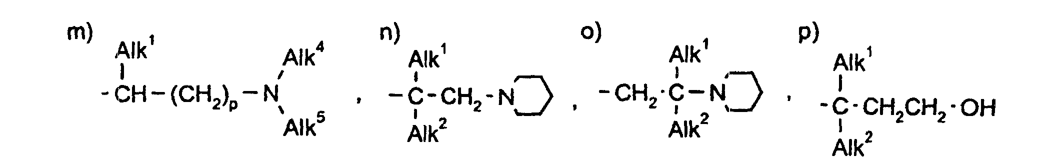 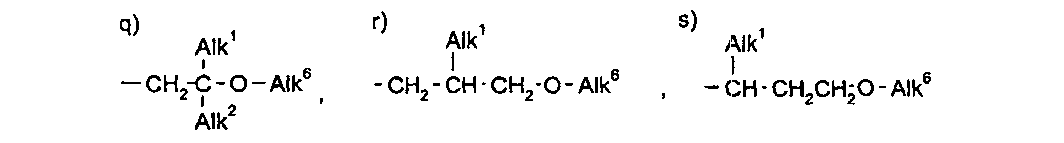 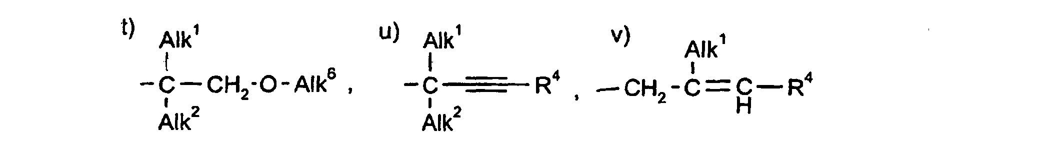 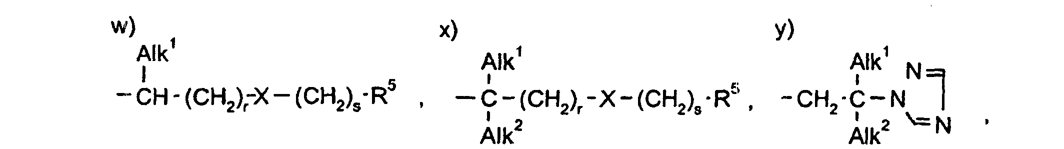
Alk¹ bis Alk⁶ unabhängig voneinander für Methyl, Ethyl und n- oder i-Propyl stehen,
Hal für Fluor und Chlor steht,
m für 1 oder 2 steht,
p für 1, 2 oder 3 steht,
r und s unabhängig voneinander für 0, 1, 2, 3 oder 4 stehen,
R³ für gegebenenfalls einfach bis dreifach, gleich oder verschieden substituiertes Phenyl oder für gegebenenfalls einfach bis zweifach, gleich oder verschieden substituiertes Pyridyl steht, wobei jeweils als Substituenten in Frage kommen:
Fluor, Chlor, Brom, Cyano, Nitro, Methyl, Ethyl, n- oder i-Propyl, t-Butyl, Trifluormethyl, Methoxy, Ethoxy, Trifluormethoxy, Trifluormethylthio, Dimethylamino, Methylethylamino und Diethylamino;
sowie für gegebenenfalls einfach bis zweifach, gleich oder verschieden substituiertes N-Morpholino, wobei als Substituenten in Frage kommen:
Methyl, Ethyl, Methoxy und Trifluormethyl,
R⁴ für gegebenenfalls einfach bis dreifach, gleich oder verschieden substituiertes Phenyl oder für gegebenenfalls einfach bis zweifach, gleich oder verschieden substituiertes Thienyl steht, wobei jeweils als Substituenten in Frage kommen:
Fluor, Chlor, Brom, Cyano, Nitro, Methyl, Ethyl, n- oder i-Propyl, t-Butyl, Trifluormethyl, Methoxy, Ethoxy, Trifluormethoxy, Trifluormethylthio, Dimethylamino, Methylethylamino und Diethylamino,
R⁵ für jeweils gegebenenfalls einfach bis dreifach, gleich oder verschieden substituiertes Phenyl und Naphthyl oder für gegebenenfalls einfach bis zweifach, gleich oder verschieden substituiertes Pyridyl steht, wobei jeweils als Substituenten in Frage kommen:
Fluor, Chlor, Brom, Cyano, Nitro, Methyl, Ethyl, n- oder i-Propyl, t-Butyl, Trifluormethyl, Methoxy, Ethoxy, Trifluormethoxy, Trifluormethylthio, Dimethylamino, Methylethylamino und Diethylamino, und
X für Sauerstoff, Schwefel, N-Methyl, N-Ethyl, N-n-Propyl und N-i-Propyl steht.

3. Verfahren zur Herstellung von substituierten Tetrahydro-5-nitro-pyrimidinen der Formel (I) gemäß Anspruch 1, **dadurch gekennzeichnet, dass** man Nitromethylen-Derivate der Formel (II) 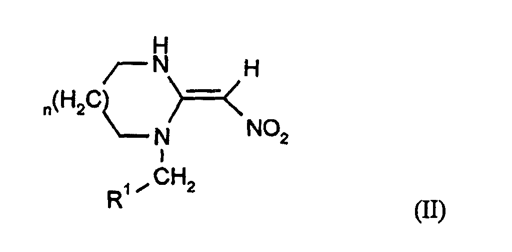 in welcher
R¹ und n die in Anspruch 1 angegebene Bedeutung haben,
mit Aminen der Formel (III)
H₂N-R² (III)
in welcher
R² die in Anspruch 1 angegebene Bedeutung hat,
in Gegenwart von mindestens der zweifachen molaren Menge Formaldehyd, gegebenenfalls in Gegenwart eines sauren Katalysators und gegebenenfalls in Gegenwart eines Verdünnungsmittels umsetzt.

4. Schädlingsbekämpfungsmittel, **gekennzeichnet durch** einen Gehalt an mindestens einem Tetrahydro-5-nitro-pyrimidin der Formel (I), gemäß Anspruch 1.

5. Verwendung von Tetrahydro-5-nitro-pyrimidinen der Formel (I) gemäß Anspruch 1 zur Bekämpfung von Schädlingen.

6. Verfahren zur Bekämpfung von Schädlingen, **dadurch gekennzeichnet, dass** man Tetrahydro-5-nitro-pyrimidine der Formel (I) gemäß Anspruch 1 auf Schädlinge und/oder ihren Lebensraum einwirken lässt.

7. Verfahren zur Herstellung von Schädlingsbekämpfungsmitteln, **dadurch gekennzeichnet, dass** man Tetrahydro-5-nitro-pyrimidine der Formel (I) gemäß Anspruch 1 mit Streckmitteln und/oder oberflächenaktiven Mitteln vermischt.

8. Verwendung von Tetrahydro-5-nitro-pyrimidinen der Formel (I) gemäß Anspruch 1 zur Herstellung von Schädlingsbekämpfungsmitteln.

## Claims

1. Substituted tetrahydro-5-nitro-pyrimidines of the formula (I) 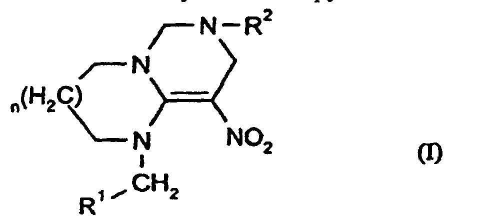 in which
n represents 0 or 1,
R¹ represents pyridyl which is optionally substituted from one to three times by identical or different substituents or represents thiazolyl which is optionally substituted one or two times by identical or different substituents, suitable substituents in each case being:
halogen, cyano, nitro, C₁-C₄-alkyl, C₁-C₄-alkoxy, C₁-C₄-alkylthio, C₁-C₄-halogenoalkyl, C₁-C₄-halogenoalkoxy and C₁-C₄-halogenoalkylthio,
R² represents one of the following groups: 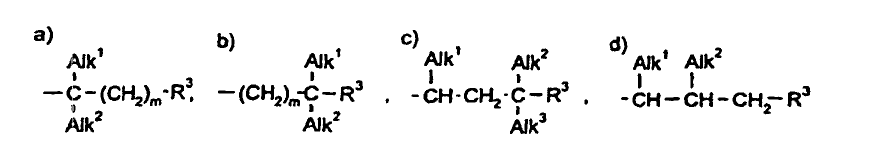 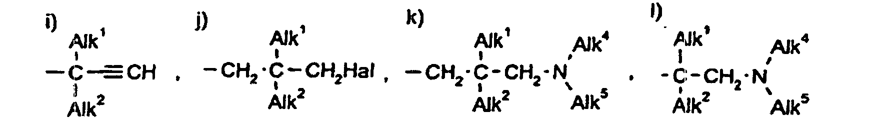 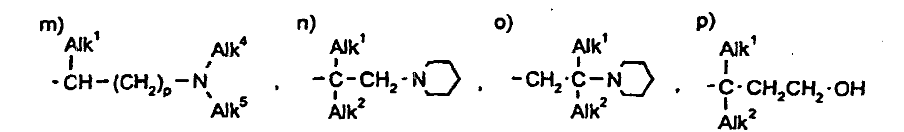 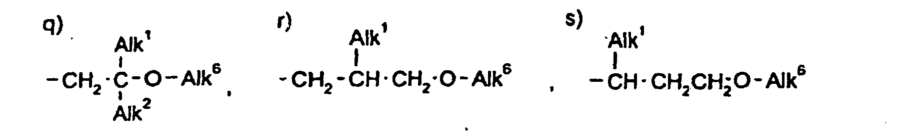 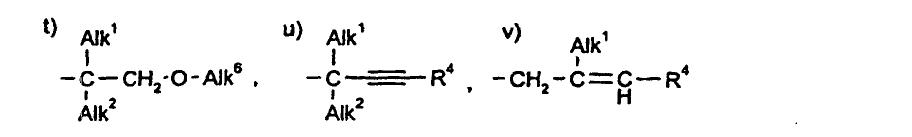 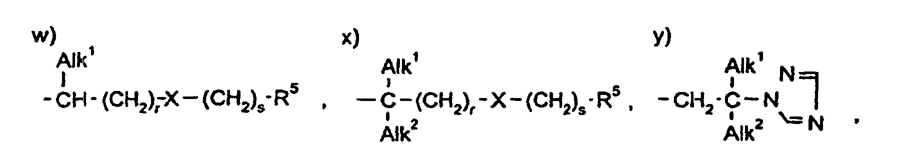
where
Alk¹ to Alk⁶ independently of one another represent C₁-C₄-alkyl,
Hal represents fluorine, chlorine or bromine,
m represents 1 or 2,
p represents 1, 2 or 3,
r and s independently of one another represent 0, 1, 2, 3 or 4,
R³ represents phenyl which is optionally substituted from one to five times by identical or different substituents or represents pyridyl which is optionally substituted from one to four times by identical or different substituents, suitable substituents in each case being: halogen, cyano, nitro, C₁-C₄-alkyl, C₁-C₄-alkoxy, C₁-C₄-alkylthio, C₁-C₄-halogenoalkyl, C₁-C₄-halogenoalkoxy, C₁-C₄-halogenoalkylthio, amino, C₁-C₄-alkylamino and di(C₁-C₄-alkyl)amino;
and also represents N-morpholino which is optionally substituted one or two times by identical or different substituents, suitable substituents being:
halogen, C₁-C₄-alkyl, C₁-C₄-alkoxy and C₁-C₄-halogenoalkyl,
R⁴ represents phenyl which is optionally substituted from one to five times by identical or different substituents or represents thienyl which is optionally substituted from one to three times by identical or different substituents, suitable substituents in each case being:
halogen, cyano, nitro, C₁-C₄-alkyl, C₁-C₄-alkoxy, C₁-C₄-alkylthio, C₁-C₄-halogenoalkyl, C₁-C₄-halogenoalkoxy, C₁-C₄-halogenoalkylthio, amino, C₁-C₄-alkylamino and di(C₁-C₄-alkyl)amino,
R⁵ represents phenyl which is optionally substituted from one to five times by identical or different substituents, naphthyl which is optionally substituted from one to three times by identical or different substituents or represents pyridyl which is optionally substituted from one to four times by identical or different substituents, suitable substituents in each case being:
halogen, cyano, nitro, C₁-C₄-alkyl, C₁-C₄-alkoxy, C₁-C₄-alkylthio, C₁-C₄-halogenoalkyl, C₁-C₄-halogenoalkoxy, C₁-C₄-halogenoalkylthio, amino, C₁-C₄-alkylamino and di(C₁-C₄-alkyl)amino and
X represents oxygen, sulphur or the group NAlk⁷, where
Alk⁷ represents C₁-C₄-alkyl.

2. Compounds of the formula (I) according to Claim 1, in which
n represents 0 or 1,
R¹ represents pyridyl which is optionally substituted one or two times by identical or different substituents or represents optionally monosubstituted thiazolyl, suitable substituents in each case being:
fluorine, chlorine, bromine, cyano, nitro, methyl, ethyl, methoxy, methylthio, trifluoromethyl, trifluoromethoxy and trifluoromethylthio,
R² represents one of the following groups: 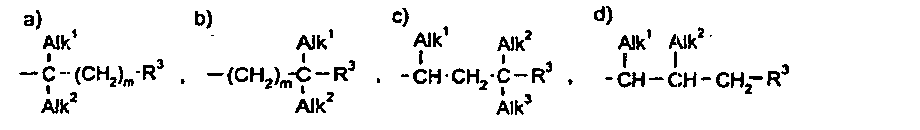 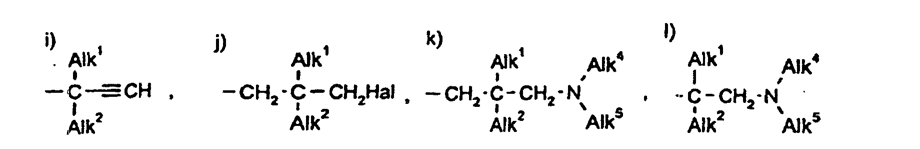 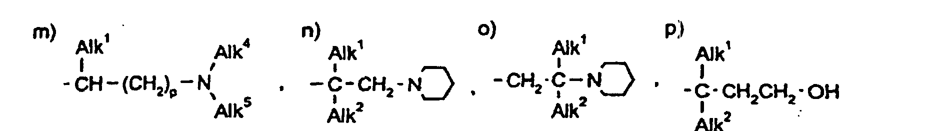 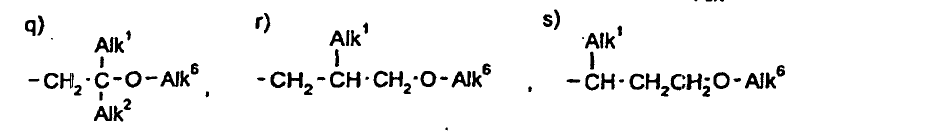 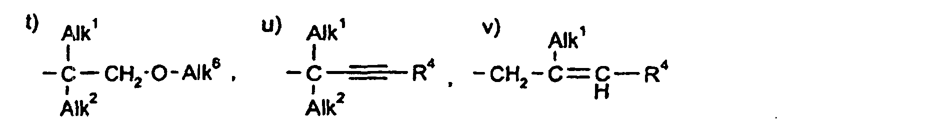 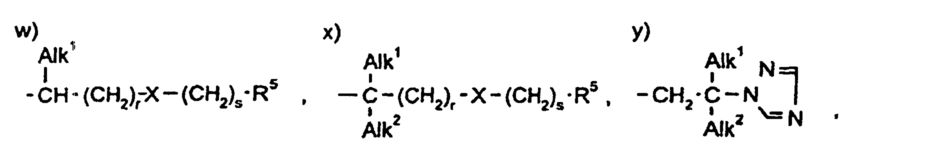
Alk¹ to Alk⁶ independently of one another represent methyl, ethyl and n- or isopropyl,
Hal represents fluorine and chlorine,
m represents 1 or 2,
p represents 1, 2 or 3,
r and s independently of one another represent 0, 1, 2, 3 or 4,
R³ represents phenyl which is optionally substituted from one to three times by identical or different substituents or represents pyridyl which is optionally substituted one or two times by identical or different substituents, suitable substituents in each case being:
fluorine, chlorine, bromine, cyano, nitro, methyl, ethyl, n- or isopropyl, t-butyl, trifluoromethyl, methoxy, ethoxy, trifluoromethoxy, trifluoromethylthio, dimethylamino, methylethylamino and diethylamino;
and also represents N-morpholino which is optionally substituted one or two times by identical or different substituents, suitable substituents being:
methyl, ethyl, methoxy and trifluoromethyl,
R⁴ represents phenyl which is optionally substituted from one to three times by identical or different substituents or represents thienyl which is optionally substituted one or two times by identical or different substituents, suitable substituents in each case being:
fluorine, chlorine, bromine, cyano, nitro, methyl, ethyl, n- or isopropyl, t-butyl, trifluoromethyl, methoxy, ethoxy, trifluoromethoxy, trifluoromethylthio, dimethylamino, methylethylamino and diethylamino,
R⁵ reprsents phenyl and naphthyl, each of which is optionally substituted from one to three times by identical or different substituents, or represents pyridyl which is optionally substituted one or two times by identical or different substituents, suitable substituents in each case being:
fluorine, chlorine, bromine, cyano, nitro, methyl, ethyl, n- or isopropyl, t-butyl, trifluoromethyl, methoxy, ethoxy, trifluoromethoxy, trifluoromethylthio, dimethylamino, methylethylamino and diethylamino, and
X represents oxygen, sulphur, N-methyl, N-ethyl, N-n-propyl and N-isopropyl.

3. Process for the preparation of substituted tetrahydro-5-nitro-pyrimidines of the formula (I) according to Claim 1, **characterized in that** nitromethylene derivatives of the formula (II) 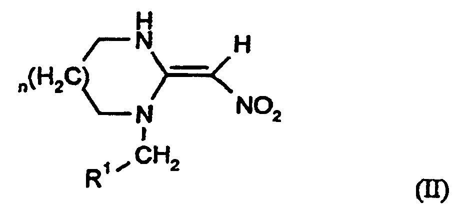 in which
R¹ and n have the meaning given in Claim 1
are reacted with amines of the formula (III)
H₂N-R² (III)
in which
R² has the meaning given in Claim 1
in the presence of at least twice the molar quantity of formaldehyde, optionally in the presence of an acidic catalyst and optionally in the presence of a diluent.

4. Composition for combating pests, **characterized by** a content of at least one tetrahydro-5-nitro-pyrimidine of the formula (I) according to Claim 1.

5. Use of tetrahydro-5-nitro-pyrimidines of the formula (I) according to Claim 1 for combating pests.

6. Method of combating pests, **characterized in that** tetrahydro-5-nitro-pyrimidines of the formula (I) according to Claim 1 are caused to act on pests and/or their habitat.

7. Process for the production of compositions for combating pests, **characterized in that** tetrahydro-5-nitro-pyrimidines of the formula (I) according to Claim 1 are mixed with extenders and/or surface-active agents.

8. Use of tetrahydro-5-nitro-pyrimidines of the formula (I) according to Claim 1 for the production of compositions for combating pests.

## Revendications

1. Tétrahydro-5-nitropyrimidines substituées de formule (I) 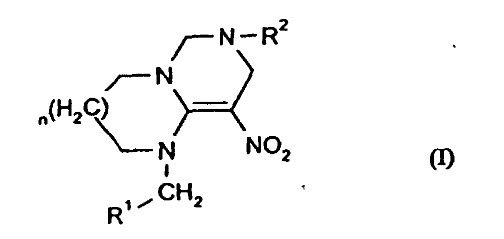 dans laquelle
n a la valeur 0 ou 1,
R¹ est un reste pyridyle éventuellement substitué une à trois fois identiques ou différentes ou un reste thiazolyle éventuellement substitué une ou deux fois identiques ou différentes, en considérant dans chaque cas les substituants suivants :
halogène, cyano, nitro, alkyle en C₁ à C₄, alkoxy en C₁ à C₄, alkylthio en C₁ à C₄, halogénalkyle en C₁ à C₄, halogénalkoxy en C₁ à C₄ et halogénalkylthio en C₁ à C₄,
R² représente l'un des groupements suivants : 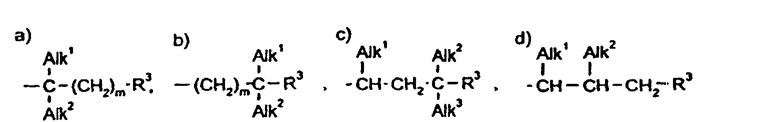 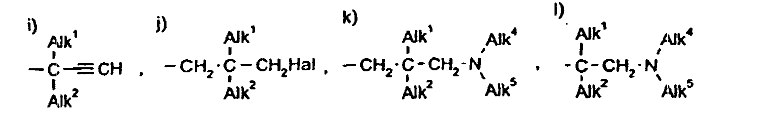 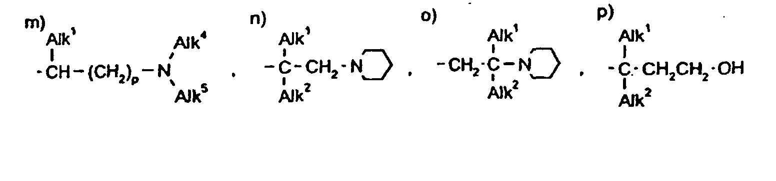 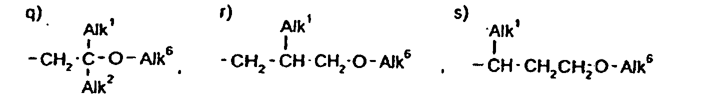 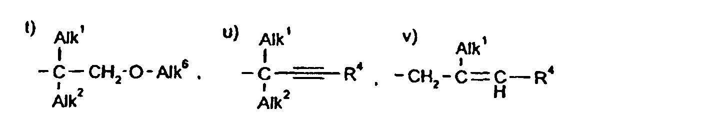 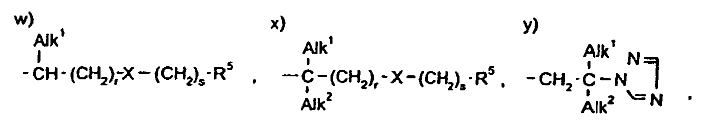
où
Alk¹ à Alk⁶ représentent indépendamment les uns des autres un reste alkyle en C₁ à C₄,
Hal représente le fluor, le chlore ou le brome,
m a la valeur 1 ou 2,
p a la valeur 1, 2 ou 3,
r et s ont, indépendamment l'un de l'autre, la valeur 0, 1, 2, 3 ou 4,
R³ est un reste phényle éventuellement substitué une à cinq fois identiques ou différentes, ou un reste pyridyle éventuellement substitué une à quatre fois identiques ou différentes, en considérant dans chaque cas les substituants :
halogène, cyano, nitro, alkyle en C₁ à C₄, alkoxy en C₁ à C₄, alkylthio en C₁ à C₄, halogénalkyle en C₁ à C₄, halogénalkoxy en C₁ à C₄, halogénalkylthio en C₁ à C₄, amino, alkylamino en C₁ à C₄ et di(alkyle en C₁ à C₄) amino ;
ainsi qu'un reste N-morpholino éventuellement substitué une ou deux fois identiques ou différentes, en considérant les substituants suivants :
halogène, alkyle en C₁ à C₄, alkoxy en C₁ à C₄ et halogénalkyle en C₁ à C₄,
R⁴ est un reste phényle éventuellement substitué une à cinq fois identiques ou différentes ou un reste thiényle éventuellement substitué une à trois fois identiques ou différentes, en considérant dans chaque cas les substituants suivants :
halogène, cyano, nitro, alkyle en C₁ à C₄, alkoxy en C₁ à C₄, alkylthio en C₁ à C₄, halogénalkyle en C₁ à C₄, halogénalkoxy en C₁ à C₄, halogénalkylthio en C₁ à C₄, amino, alkylamino en C₁ à C₄ et di (alkyle en C₁ à C₄)amino,
R⁵ est un reste phényle éventuellement substitué une à cinq fois identiques ou différentes, un reste naphtyle éventuellement substitué une à trois fois identiques ou différentes ou un reste pyridyle éventuellement substitué une à quatre fois identiques ou différentes, en considérant dans chaque cas les substituants suivants :
halogène, cyano, nitro, alkyle en C₁ à C₄, alkoxy en C₁ à C₄, alkylthio en C₁ à C₄, halogénalkyle en C₁ à C₄, halogénalkoxy en C₁ à C₄, halogénalkylthio en C₁ à C₄, amino, alkylamino en C₁ à C₄ et di (alkyle en C₁ à C₄) amino, et
X représente l'oxygène, le soufre ou le groupement NAlk⁷, dans lequel
Alk⁷ est un reste alkyle en C₁ à C₄.

2. Composés de formule (I) suivant la revendication 1, formule dans laquelle
n a la valeur 0 ou 1,
R¹ est un reste pyridyle éventuellement substitué une ou deux fois identiques ou différentes ou un reste thiazolyle éventuellement substitué une fois, en considérant dans chaque cas les substituants suivants :
fluor, chlore, brome, cyano, nitro, méthyle, éthyle, méthoxy, méthylthio, trifluorométhyle, trifluorométhoxy et trifluorométhylthio,
R² représente l'un des groupements suivants : 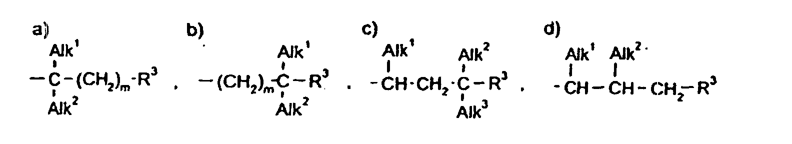 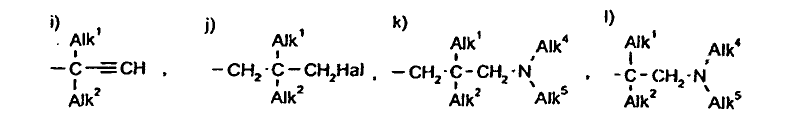 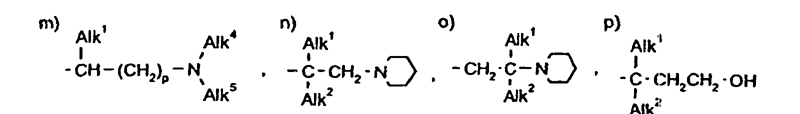 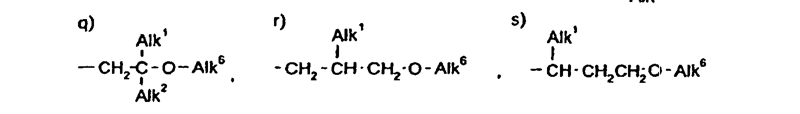 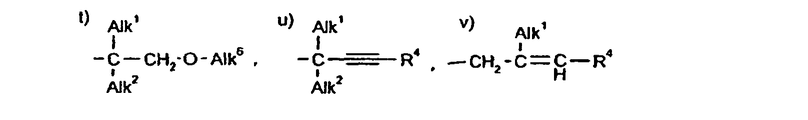 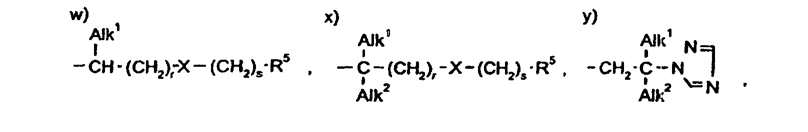
Alk¹ à Alk⁶ représentent indépendamment les uns des autres un reste méthyle, éthyle, n-propyle ou isopropyle,
Hal représente le fluor et le chlore,
m a la valeur 1 ou 2,
p a la valeur 1, 2 ou 3,
r et s ont, indépendamment l'un de l'autre, la valeur 0, 1, 2, 3 ou 4,
R³ est un reste phényle éventuellement substitué une à trois identiques ou différentes ou un reste pyridyle éventuellement substitué une ou deux fois identiques ou différentes, en considérant dans chaque cas les substituants suivants :
fluor, chlore, brome, cyano, nitro, méthyle, éthyle, n-propyle, isopropyle, tertiobutyle, trifluorométhyle, méthoxy, éthoxy, trifluorométhoxy, trifluorométhylthio, diméthylamino, méthyléthylamino et diéthylamino ;
ainsi qu'un reste N-morpholino éventuellement substitué une ou deux fois identiques ou différentes, en considérant les substituants suivants :
méthyle, éthyle, méthoxy et trifluorométhyle,
R⁴ est un reste phényle éventuellement substitué ou à trois fois identiques ou différentes ou un reste thiényle éventuellement substitué une ou deux fois identiques ou différentes, en considérant dans chaque cas les substituants suivants :
fluor, chlore, brome, cyano, nitro, méthyle, éthyle, n-propyle, isopropyle, tertiobutyle, trifluorométhyle, méthoxy, éthoxy, trifluorométhoxy, trifluorométhylthio, diméthylamino, méthyléthylamino et diéthylamino,
R⁵ représente des restes phényle et naphtyle dont chacun est éventuellement substitué une à trois identiques ou différentes ou un reste pyridyle éventuellement substitué une ou deux fois identiques ou différentes, en considérant dans chaque cas les substituants suivants :
fluor, chlore, brome, cyano, nitro, méthyle, éthyle, n-propyle, isopropyle, tertiobutyle, trifluorométhyle, méthoxy, éthoxy, trifluorométhoxy, trifluorométhylthio, diméthylamino, méthyléthylamino et diéthylamino, et
X représente l'oxygène, le soufre, les restes M-méthyle, N-éthyle, N-n-propyle et N-isopropyle.

3. Procédé de production de tétrahydro-5-nitro-pyrimidines substituées de formule (I) suivant la revendication 1, **caractérisé en ce qu'**on fait réagir des dérivés nitrométhyléniques de formule (II) 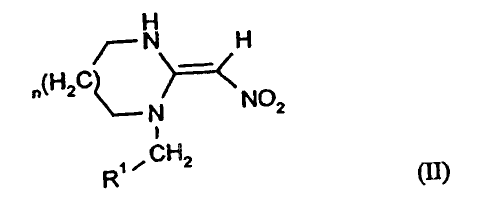 dans laquelle
R¹ et n ont la définition indiquée dans la revendication 1,
avec des amines de formule (III)
**H**_{**2**}**N-R**^{**2**} **(III)**
dans laquelle
R² a la définition indiquée dans la revendication 1,
en présence d'au moins la double quantité molaire de formaldéhyde, éventuellement en présence d'un catalyseur acide et, le cas échéant, en présence d'un diluant.

4. Compositions pesticides, **caractérisées par** une teneur en au moins une tétrahydro-5-nitropyrimidine de formule (I) suivant la revendication 1.

5. Utilisation de tétrahydro-5-nitropyrimidines de formule (I) suivant la revendication 1 pour combattre des parasites.

6. Procédé de lutte contre des parasites, **caractérisé en ce qu'**on laisse agir des tétrahydro-5-nitropyrimidines de formule (I) suivant la revendication 1, sur des parasites et/ou sur leur milieu.

7. Procédé de préparation de compositions pesticides, **caractérisé en ce qu'**on mélange des tétrahydro-5-nitropyrimidines de formule (I) suivant la revendication 1 avec des diluants et/ou des agents tensioactifs.

8. Utilisation de tétrahydro-5-nitropyrimidines de formule (I) suivant la revendication 1 pour la préparation de compositions pesticides.
